# EUROPEAN PATENT SPECIFICATION

(11) **EP 4 364 656 B1**
(45) Date of publication and mention of the grant of the patent: **08.04.2026**
(21) Application number: 22853248.7
(22) Date of filing: 02.06.2022
(51) Int. Cl.: A61B 5/145, A61B 5/15, A61B 5/151, A61B 5/155, A61B 5/00

(54) **APPLICATOR FOR TRANSCUTANEOUS SENSOR, AND APPLICATOR ASSEMBLY**
APPLIKATOR FÜR TRANSKUTANEN SENSOR UND APPLIKATORANORDNUNG
APPLICATEUR POUR CAPTEUR TRANSCUTANÉ, ET ENSEMBLE APPLICATEUR

(30) Priority: 05.08.2021 KR 20210103240
(43) Date of publication of application: 08.05.2024
(60) Divisional application: 26157729.0
(73) Proprietor: i-Sens, Inc., Seoul 06646 (KR)
(72) Inventor: CHOI, Hyun Ho, Seoul 06646 (KR); RYU, Goang Yel, Seoul 06646 (KR); WANG, Ji Hoon, Seoul 06646 (KR)
(74) Representative: BCKIP Part mbB
(86) International application number: PCT/KR2022/007868
(87) International publication number: WO 2023/013868

(56) References cited:
- EP-A1- 3 632 314
- JP-A- 2008 506 468
- KR-A- 20180 132 553
- KR-A- 20200 013 996
- KR-A- 20200 014 001
- KR-A- 20200 014 002
- KR-A- 20200 127 097
- US-A1- 2020 289 748

## Description

### TECHNICAL FIELD

The present invention relates to an applicator for a transcutaneous sensor, and more specifically, related to an applicator and an applicator assembly for inserting a transcutaneous sensor, which is inserted into the skin of a user to measure biometric information, into the skin of a user.

### BACKGROUND

With the recent advancement of medical technology, various medical devices that are attached to the body of a user have been developed and sold. Medical devices that are attached to the skin can be useful for monitoring biometric information or providing treatment by attaching them to the body of a patient with a chronic disease.

For example, chronic diseases such as diabetes require continuous management, and a body attachable unit for measuring biometric information can be used to manage blood glucose levels in diabetic patients. Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown. Further progression of diabetes leads to complications such as visual disturbances, hypertension, kidney disease, paralysis, periodontal disease, muscle spasms and neuralgia, as well as gangrene. In order to diagnose diabetes beforehand and manage to prevent the progression of diabetes into complications associated therewith, systematic blood glucose measurement and treatment should be performed.

For diabetes patients as well as people having higher than normal blood glucose, even though diabetes has not yet developed, medical device manufacturers offer a variety of blood glucose meters to measure blood glucose levels.

Glucose measuring devices may be categorized into a single time measurement type measuring a blood glucose level and collecting blood from a fingertip by a user every single time and a continuous measurement type attaching a glucose monitoring system to the belly or an arm of the user and continuously measuring blood glucose levels.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

A continuous blood glucose measurement system includes a body attachable unit that includes a transcutaneous sensor that is inserted into the skin of a user and measures blood glucose levels in the body, and a terminal that receives biometric information transmitted from the body attachable unit and outputs.

Systems for medical purposes using transcutaneous sensors are produced in many different forms by each manufacturer, and the methods of use also vary. Most systems currently manufactured and distributed are a method of attaching a disposable sensor unit to a body by an applicator. A user needs to perform several steps to attach the sensor unit to a skin using an applicator, and after attaching the sensor unit to a body, various follow-up procedures such as having to directly pull out the needle inserted into the skin along with the transcutaneous sensor are required to be performed.

For example, a user must peel off the packaging of the disposable sensor unit and accurately attach it to the applicator, and then insert the sensor unit into the applicator and operate the applicator to attach the sensor unit to a skin. Additionally, after attaching the sensor unit, there is the inconvenience of having to perform tasks such as pulling out the needle inserted into the skin and coupling the transmitter to the sensor unit.

US 2020 / 0 289 748 A1 discloses an inserter assembly comprising a first unit including a skin contacting face which surrounds an opening and a second unit housed within the first unit. The second unit comprises an infusion set base disposed within the opening and having a bottom face which is substantially level with the skin contacting face and covered at least partially with adhesive and further comprising a spring biased insertion assembly. The second unit further comprising a cannula sub assembly carried by an insertion sharp of the insertion assembly. The spring biased insertion assembly and a cannula of the cannula sub assembly are driven into skin and the cannula sub assembly is coupled into the infusion set base by an insertion spring which is released from an energy storing state after the skin has been tugged upward beyond a certain distance by the adhesive as the inserter assembly is withdrawn from the body.

### SUMMARY OF THE INVENTION

### Technical Problem

The present invention is developed in consideration of the above-mentioned points, and aims to provide an applicator and applicator assembly for a transdermal sensor which may be manufactured in a state assembled with a sensor unit including a transdermal sensor, thereby minimizing the user's additional work to insert the transdermal sensor into skin and being conveniently used when the user inserts the transdermal sensor into the skin.

Another object of the present invention is to provide an applicator and applicator assembly for a transdermal sensor that can automatically separate a protective sheet that covers and protects an adhesive layer provided at a sensor unit.

### Solution to Problem

To accomplish the purposes described above, the present invention provides an applicator in accordance with claim 1 and an applicator assembly in accordance with claim 12.

According to the present invention, an applicator for inserting a sensor for measuring biometric information into skin of a user, comprises: an applicator body; an insertion unit installed to the applicator body to move a sensor unit, which includes the sensor, a sensor unit housing to which the sensor is mounted, an adhesive layer provided at the sensor unit housing, and a protective sheet covering an adhesive layer, from a first position spaced apart from the skin of the user to a second position where the sensor is inserted into the skin of the user; a removing member to which a portion of the protective sheet is coupled and is rotatably installed to the applicator body to remove the protective sheet from the adhesive layer; and an operation member installed to the applicator body to operate the removing member by being manipulated by the user.

The removing member may be configured to be rotatable around a central rotation axis perpendicular to a movable direction of the sensor unit.

The removing member may be coupled to the protective sheet by an adhesive member.

The applicator according to the present invention comprises a removing member driver configured to provide rotary force to the removing member, wherein the operation member is configured to operate the removing member driver such that the removing member is rotated by the removing member driver.

The removing member driver may be coupled with the removing member and configured to apply elastic force to the removing member.

The applicator according to an embodiment of the present invention may comprise a stopper member installed to the applicator body to restrain movement of the removing member by being contacted with the removing member to maintain a position in which the removing member is coupled with the protective sheet in a state that a removing elastic member is elastically deformed, wherein the operation member is operated to release restraining force of the removing member caused by the stopper member.

The stopper member may be installed to be movable in a direction of intersecting with a movable direction of the sensor unit.

The applicator according to an embodiment of the present invention may comprise an elastic member configured to apply elastic force to the stopper member such that the stopper member is movable in a direction of releasing the restraining force of the removing member; and a stopper member fixing portion provided at the stopper member or the applicator body to interlock the stopper member and the applicator body to maintain a position in which the stopper member restrains movement of the removing member in a state that the elastic member is elastically deformed, wherein the operation member is operated to release fixing force of the stopper member caused by the stopper member fixing portion.

The insertion unit may comprise a shuttle installed to be movable from the first position to the second position together with the sensor unit, a needle coupled with the shuttle to be movable from the first position to the second position together with the shuttle, and separably coupled to the sensor unit to be inserted into the skin of the user together with the sensor, and a shuttle driver configured to provide moving force to the shuttle in a direction of moving the first position to the second position, and movement of the shuttle is restrained by the stopper member at the first position, and the shuttle is movable to the second position by the shuttle driver by releasing restraining force of the stopper member according to movement of the stopper member.

The stopper member may be configured to release restraining force applied to the removing member first, and after completing a separation operation of the protective film by the removing member, release restraining force applied to the shuttle.

The removing member may be configured to complete a separation operation of the protective sheet before the sensor unit is moved from the first position.

According to an embodiment of the present invention, the applicator body is separably coupled to a base unit including a base unit housing, and an adhesive portion provided at the base unit housing to be attached to the skin of the user.

Additionally, to accomplish the purposes described above, according to the present invention, an applicator assembly comprises: a sensor unit including a sensor, a sensor unit housing to which the sensor is mounted, an adhesive layer provided at the sensor unit housing, and a protective sheet covering the adhesive layer; and an applicator according to any one of the embodiments described above.

The applicator according to an embodiment of the present invention may comprise a base unit including a base unit housing to which the sensor unit housing is coupled and an adhesive portion provided at the base unit housing to be attachable to the skin of the user, and spaced apart from the sensor unit and separably coupled to the applicator body, and the sensor unit housing is configured to be attachable to the base unit housing by the adhesive layer at the second position.

### Advantageous Effects of Invention

According to the present invention, by manufacturing a sensor unit in a state assembled inside an applicator, a user's additional work to attach the sensor unit to the skin of a user may be minimized, and the sensor unit may be attached to the skin of the user only by simply operating the applicator.

Additionally, according to the present invention, by covering and protecting an adhesive layer of a sensor unit with a protective sheet, it is possible to prevent the problem of deterioration of the adhesiveness of the adhesive layer before inserting the sensor into the skin of a user.

Additionally, according to the present invention, a protective sheet that covers and protects an adhesive layer of a sensor unit can be automatically separated by simply operating an applicator without the need for the user to remove the protective sheet. Therefore, no additional work is required to separate the adhesive layer of the sensor unit, and convenience of use may be improved.

### BRIEF DESCRIPTION OF THE DRAWINGS

FIG. 1 is a perspective view showing an applicator assembly according to an embodiment of the present invention.
FIG. 2 shows a body attachable unit attached to skin of a user.
FIG. 3 is a perspective view showing a body attachable unit.
FIGS. 4 and 5 are exploded perspective views for showing a sensor unit.
FIG. 6 shows a state in which a needle is coupled to a sensor unit.
FIG. 7 is a perspective view for showing a sensor unit and a base unit before they are coupled.
FIG. 8 is a cross-sectional view for showing a sensor unit and a base unit before they are coupled.
FIG. 9 is a cross-sectional view showing a sensor unit and a base unit combined to form a body attachable unit.
FIG. 10 is a cross-sectional view taken along line I-I in FIG. 1.
FIG. 11 is a cross-sectional view taken along line II-II in FIG. 1.
FIG. 12 is an exploded perspective view of the applicator according to an embodiment of the present invention.
FIG. 13 shows a view in which a locking hook is separated from a base frame.
FIG. 14 shows an exploded view of an insertion unit assembly.
FIG. 15 shows a view before a sensor unit is coupled to an insertion unit and another view after the sensor unit is coupled to the insertion unit.
FIG. 16 shows a view in which an insertion unit assembly is coupled to an applicator body.
FIG. 17 shows a sensor insertion operation of an insertion unit.
FIG. 18 shows a view in which a carrier and a needle of a needle assembly are separated.
FIG. 19 shows a cross-sectional view in which a carrier and a needle of a needle assembly are coupled.
FIGS. 20 and 21 are exploded views of a portion of an applicator according to an embodiment of the present invention.
FIG. 22 shows a process of moving a stopper member from a third position to a fourth position.
FIG. 23 shows a view in which a removing member is separated from a protective sheet of a sensor unit.
FIG. 24 shows a view in which a removing member is coupled with a protective sheet of a sensor unit.
FIG. 25 shows a process in which a removing unit is operated to separate a protective sheet of a sensor unit.
FIGS. 26 to 28 show a process in which a sensor of a sensor unit is inserted into skin of a user by an applicator according to an embodiment of the present invention.
FIG. 29 shows a process in which a removing unit separates a protective sheet of a sensor unit in a different way.
FIG. 30 is a cross-sectional view showing an applicator assembly according to another embodiment of the present invention.
FIG. 31 shows a sensor unit of an applicator assembly shown in FIG. 30.
FIGS. 32 and 33 show a sensor unit and a base unit according to other embodiments of the present invention.

### DETAILED DESCRIPTION OF EMBODIMENTS

Hereinafter, the applicator and applicator assembly for a transcutaneous sensor according to the present invention will be described in detail with reference to the drawings.

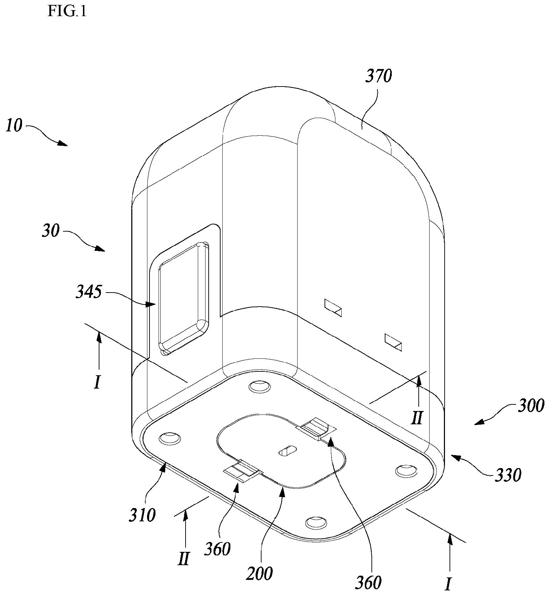

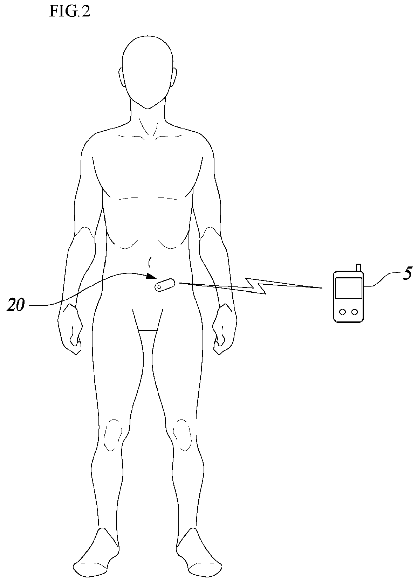

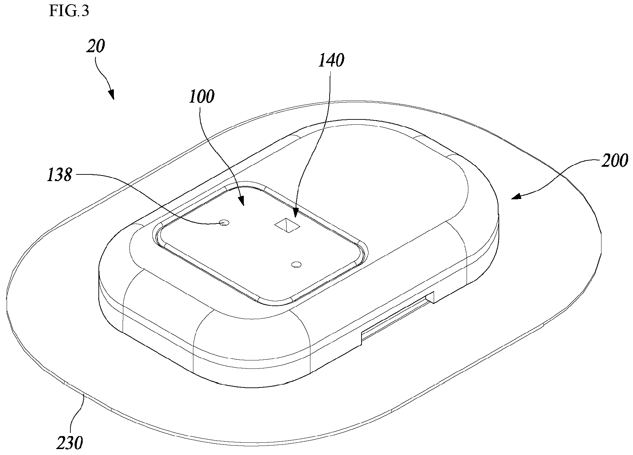

FIG. 1 is a perspective view showing an applicator assembly according to an embodiment of the present invention, FIG. 2 is a perspective view showing the body attachable unit attached to the skin of a user, and FIG. 3 is a perspective view showing the body attachable unit.

An applicator assembly (10) according to the present invention includes a sensor unit (100) including a sensor (110) that is inserted into the skin of a user and measures biometric information, and a base unit (200) constituting body attachable unit (20) with sensor unit (100), and an applicator (30) for attaching the body attachable unit (20) to the skin of a user. The applicator assembly (10) may be provided to a user with the sensor unit (100) and the base unit (200) mounted separately on the applicator (30). The sensor unit (100) and the base unit (200) can be automatically assembled and attached to the skin of a user by the operation of the applicator (30).

As shown in FIG. 2, the body attachable unit (20) can measure biometric information by being attached to the skin of a user, and wirelessly transmit the measured data to an external terminal (5). The external terminal (5) may be a variety of devices capable of receiving measurement data from the body attachable unit (20), such as a portable terminal, a medical device, a PC, or a server. Biometric information that the body attachable unit (20) can measure is not limited to specific information. As an exemplary embodiment, the body attachable unit (20) may periodically measure blood glucose level for a user and transmit the blood glucose measurement information to the external terminal (5).

As shown in FIG. 3, the body attachable unit (20) includes the sensor unit (100) for measuring the biometric information of a user, and the base unit (200) that is attached to the skin of a user and coupled to the sensor unit (100). The base unit (200) may have electronic components installed therein. This base unit (200) is electrically connected to the sensor unit (100) and can process biometric information measured by the sensor unit (100) and transmit it to the external terminal (5). The base unit (200) may be configured to simply support the sensor unit (100) so that it is not separated from the skin of a user. In this case, a separate electronic unit that can process biometric information measured by the sensor unit (100) and transmit it to the external terminal (5) may be detachably coupled to the base unit (200).

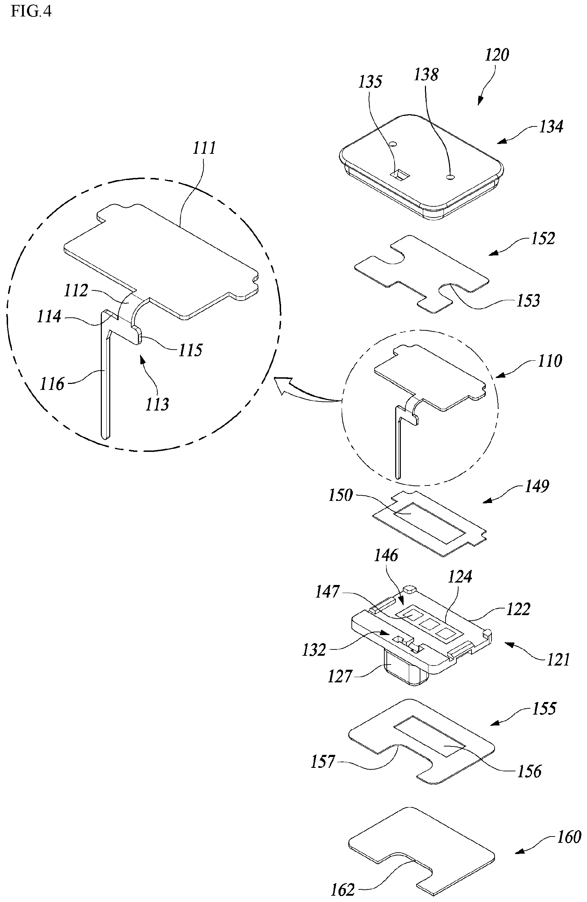

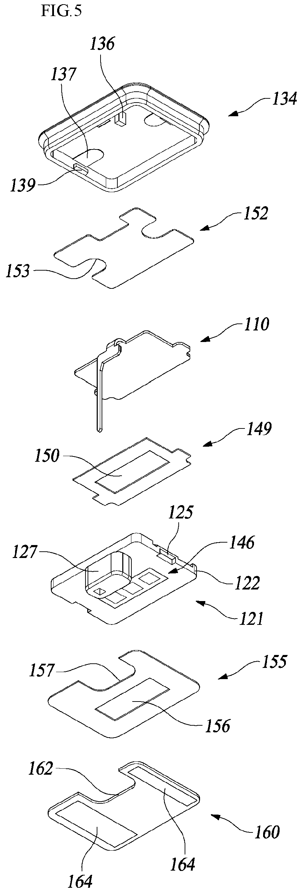

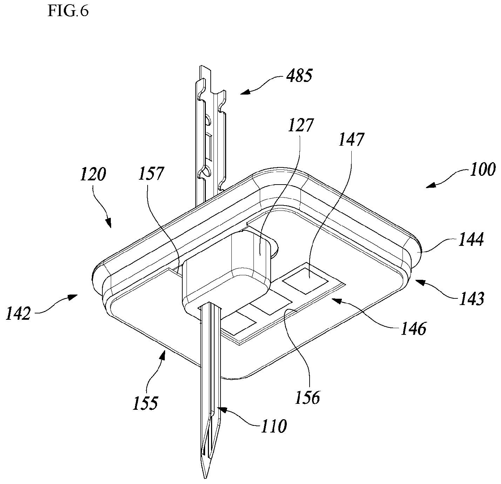

As shown in FIGS. 4 to 9, the sensor unit (100) includes the sensor (110) inserted into the skin of a user, a sensor unit housing (120) to which the sensor (110) is coupled, a sensor unit-electrical contact portion(146) electrically connected to the sensor (110) coupling to the sensor unit housing(120), an adhesive layer (155) provided on the sensor unit housing (120) so as to be attached to the base unit (200), a protective sheet (160) covering the adhesive layer (155).

The sensor (110) includes a sensor body (111), a connection portion (112) connected to one side of the sensor body (111), a middle portion (113) connected to the sensor body (111) through the connection portion (112), and an insertion portion (116) connected to the middle portion (113) so as to be inserted into the skin of a user. The sensor body (111) is disposed inside the sensor unit housing (120) so as to be in contact with the sensor unit-electrical contact portion (146). The sensor body (111) may be provided with an electrode electrically connected to the sensor unit-electrical contact portion (146). The connection portion (112) extends from the edge of the sensor body (111) and connects the sensor body (111) and the middle portion (113) to lie on different planes. The sensor (110) may be manufactured in a flat shape in which the sensor body (111) lies on the same plane as the middle portion (113) and the insertion portion (116), and then manufactured in a form in which the connection portion (112) is bent and deformed so that the sensor body (111) lies on a different plane from the middle portion (113) and the insertion portion (116).

The middle portion (113) and the insertion portion (116) are connected to the sensor body (111) so as to lie on a different plane from the sensor body (111). In the drawing, the middle portion (113) and the insertion portion (116) are shown to be disposed on the same plane, and the middle portion (113) is disposed perpendicular to the sensor body (111), but the angle between the middle portion (113) and the sensor body (111) or the angle between the insertion portion (116) and the sensor body (111) can be changed in various ways.

The middle portion (113) includes a first extension portion (114) that protrudes to one side of the connection portion (112), and a second extension portion (115) that protrudes from the connection portion (112) in a direction opposite to the direction in which the first extension portion (114) protrudes from the connection portion (112). The insertion portion (116) may be connected to the first extension portion (114) and inserted into the skin of a user. The insertion portion (116) has a relatively thin and long shape so as to be smoothly inserted into the skin of a user. The connection portion (112), the middle portion (113), and the insertion portion (116) may be provided with a conductive trace electrically connected to the electrode of the sensor body (111). The sensor (110) may be coupled to the sensor unit housing (120) so that the sensor body (111), the connection portion (112) and the middle portion (113) are located inside the sensor unit housing (120) and only the insertion portion (116) protrudes from the sensor unit housing (120).

The sensor unit housing (120) includes a housing base (121) and a housing cap (134) that covers the top of the housing base (121). A space is provided inside the sensor unit housing (120) to accommodate the sensor (110), and a housing opening (140) into which a needle (450) for inserting the sensor (110) into the skin of a user is inserted is formed in the middle of the sensor unit housing (120) to penetrate the sensor unit housing (120) in the thickness direction.

The housing base (121) includes a base portion (122) supporting the sensor body (111) of the sensor (110) and a boss (127) protruding from the surface of the base portion (122). On one side of the base portion (122), a through hole (124) is formed to penetrate the base portion (122). A sensor unit-electrical contact portion (146) is coupled to the through hole (124). A detent groove (125) is provided at the edge of the base portion (122). The boss (127) protrudes from one of the surfaces on both sides of the base portion (122) where the sensor body (111) is not placed. The boss (127) is provided with a housing base hole (132). The housing base hole (132) extends from the end of the boss (127) to one surface of the base portion (122). The insertion portion (116) of the sensor (110) and needle (485) may be inserted into the housing base hole (132).

The housing cap (134) is coupled to the housing base (121) and covers the sensor body (111) of the sensor (110) placed on the housing base (121) and the upper part of the housing base (121). A housing cap hole (135) is formed in the middle of the housing cap (134) to penetrate the housing cap (134) in the thickness direction. The housing cap hole (135) is connected to the housing base hole (132) of the housing base (121) to form the housing opening (140). The needle (450) for inserting the sensor (110) into the skin of a user is inserted along with the sensor (110) into the housing opening (140). A sensor unit groove (138) is formed on the upper surface of the housing cap (134). Additionally, the housing cap (134) is provided with a detent protrusion (139) corresponding to the detent groove (125) of the housing base (121). The housing cap (134) may be coupled to the housing base (121) by inserting the detent protrusion (138) into the detent groove (125) and engaging the housing base (121). A protrusion (137) and a retention protrusion (136) are provided on the inner surface of the housing cap (134) facing the housing base (121). The protrusion (137) and the retention protrusion (136) protrude toward the housing base (121). The protrusion (137) and the retention protrusion (136) may contact the sensor (110) to prevent movement of the sensor (110) and stably fix the sensor (110) to the sensor unit housing (120). The protrusion (137) may contact the sensor body (111) of the sensor (110) and press the sensor body (111) toward the housing base (121). Therefore, the sensor body (111) can remain firmly fixed to the housing base (121) without being lifted from the housing base (121).

The protrusion (137) may contact the sensor body (111) and press the sensor body (111) toward the housing base (121). Therefore, the sensor body (111) may remain firmly fixed to the housing base (121) without being lifted from the housing base (121). The retention protrusion (136) is inserted into the housing base hole (132) and comes into contact with the sensor (110). That is, the retention protrusion (136) may be arranged such that its end faces the second extension (115) of the middle portion (113) inserted into the housing base hole (132) to restrain the movement of the middle portion (113) so that the middle portion (113) of the sensor (110) is not lifted. As the action of the retention protrusion (136), the insertion portion (116) of the sensor (110) can be stably maintained in a protruded state from the sensor unit housing (120) by a certain length. And, due to the action of the retention protrusion (136), the insertion portion (116) of the sensor (110) may not retreat from the skin of a user while being inserted into the skin of a user, and remain inserted at a certain depth into the skin of a user. In addition, after the insertion portion (116) is inserted into the skin of a user with the needle 485, the retention protrusion (136) prevents the middle portion (113) from moving while the needle (485) exits the skin of a user, so that the insertion portion (116) cannot retreat in the same direction as the needle (485). Accordingly, it is possible to prevent a problem in which the insertion portion (116) inserted into the skin of a user moves in the direction of exiting the skin of a user in the process of the needle (485) coming out of the skin of a user. Additionally, the insertion portion (116) partially comes out of the skin, thereby preventing the measurement accuracy of the sensor (110) from being reduced.

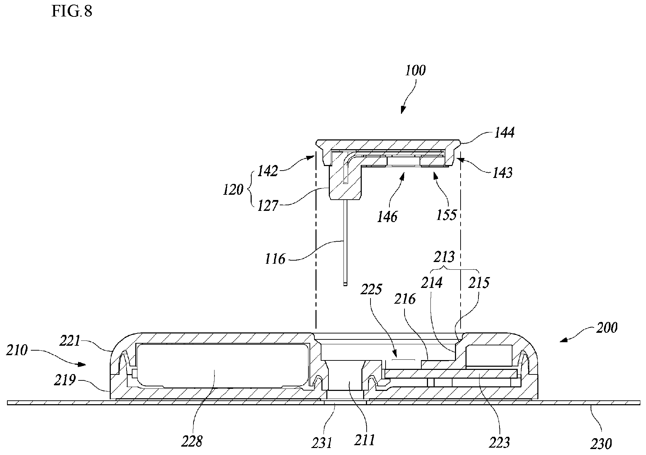

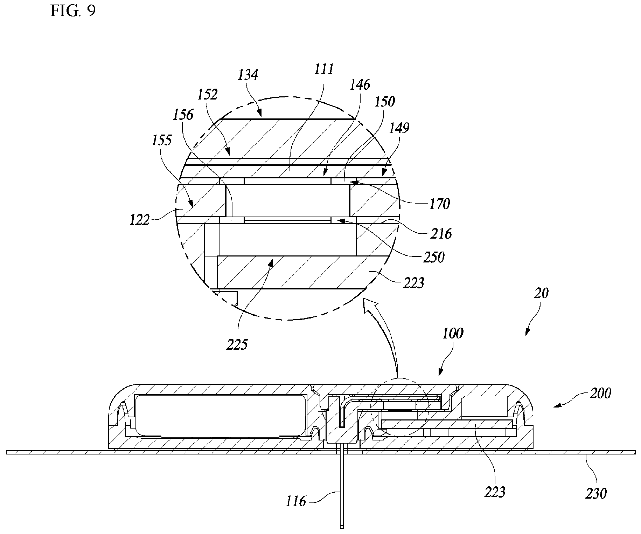

The housing cap (134) forms the housing body (142) together with the base portion (122). That is, the sensor unit housing (120) may be configured to include a housing body (142) and a boss (127) protruding from one side of the housing body (142). As shown in FIGS. 8 and 9, the housing body (142) is shaped to fit into a base unit recess (213) of the base unit (200). The housing body (142) includes a body portion (143) provided with a boss (127) and a cover portion (144) that is wider than the body portion (143).

The sensor unit housing (120) is not limited to the configuration shown, and may be changed to various other configurations on which the sensor (110) may be mounted and coupled to the base unit (200). That is, the sensor unit housing (120) includes a housing base (121) and the housing cap (134), but it can be changed to various configurations other than a configuration including a housing body (142) and a boss (127) protruding from the housing body (142).

The sensor unit-electrical contact portion (146) is disposed in the sensor unit housing (120) to be electrically connected to the sensor body (111). The sensor unit-electrical contact portion (146) is inserted into the through hole (124) of the base portion (122) and is disposed to vertically penetrate the base portion (122). Some part of the sensor unit-electrical contact portion (146) is exposed to one surface of the base portion (122) where the sensor body (111) is located, and another part is exposed to the other side of the base portion (122), so that the sensor (110) and a base unit-electrical contact portion (225) of the base unit (200) can be electrically connected.

The sensor adhesive portion (149) is disposed between the housing base (121) and the sensor body (111) to attach the sensor body (111) to the housing base (121). The sensor adhesive portion (149) has adhesive properties on both sides. That is, one side of the sensor adhesive portion (149) is adhered to the housing base (121), and the other surface of the sensor adhesive portion (149) is adhered to the sensor body (111). A sensor adhesive portion opening (150) is formed in the middle of the sensor adhesive portion (149) to penetrate the sensor adhesive portion (149) in the thickness direction. The sensor body (111) is in contact with the sensor unit-electrical contact portion (146) through the sensor adhesive portion opening (150). Therefore, the sensor adhesive portion (149) seals between the sensor body (111) and the sensor unit-electrical contact portion (146), thereby preventing moisture or foreign substances from entering an electrical connection portion (170) between the sensor body (111) and the sensor unit electrical contact portion (146).

The shape of the sensor adhesive portion (149) is not limited to that shown and can be changed in various ways.

The adhesive pad (152) is disposed between the housing cap (134) and the sensor body (111) to attach the sensor body (111) to the housing cap (134). The adhesive pad (152) has adhesive properties on both sides. That is, one side of the adhesive pad (152) is adhered to the housing cap (134), and the other side of the adhesive pad (152) is adhered to the sensor body (111). An adhesive pad groove (153) is formed in the adhesive pad (152) to penetrate the adhesive pad (152) in the thickness direction. The protrusion (137) of the housing cap (134) contacts the sensor body (111) through the adhesive pad groove (153), thereby pressing the sensor body (111) toward the housing base (121).

The shape of the adhesive pad (152) is not limited to that shown and can be changed in various ways.

An adhesive layer (155) is disposed on the surface of the housing base (121). The adhesive layer (155) may be in the form of a double-sided tape with adhesive properties on both sides. One side of the adhesive layer (155) is adhered to the housing base (121), and the other side of the adhesive layer (155) is covered with a protective sheet (160). The adhesive layer (155) may be attached to the base unit (200) after the protective sheet (160) is separated. An adhesive layer hole (156) is formed in the middle portion of the adhesive layer (155) to penetrate the adhesive layer (155) in the thickness direction. The sensor unit electrical contact portion (146) may contact the base unit electrical contact portion (225) of the base unit (200) through the adhesive layer hole (156). Therefore, the adhesive layer (155) seals between the sensor unit electrical contact portion (146) and the base unit electrical contact portion (225), thereby preventing moisture or foreign substances from entering the electrical connection portion (250) between the sensor unit (100) and the base unit (200). An adhesive layer opening (157) is formed on one side of the adhesive layer (155) to penetrate the adhesive layer (155) in the thickness direction. The adhesive layer (155) is attached to the housing base (121) such that the sensor unit electrical contact portion (146) is located inside the adhesive layer hole (156) and the boss (127) is inserted into the adhesive layer opening (157).

The shape of the adhesive layer (155) is not limited to that shown and can be changed in various ways.

The protective sheet (160) covers and protects the adhesive layer (155). The protective sheet (160) is made of a material that is detachably attached to the adhesive layer (155). If the adhesive layer (155) is left exposed to the air for a long time, the adhesiveness of the adhesive layer (155) may deteriorate. The protective sheet (160) covers the adhesive layer (155) to prevent the problem of reducing of adhesiveness of the adhesive layer (155), and it facilitates handling of the sensor unit (100) by an operator during the manufacturing process of the sensor unit (100) or the process of assembling the sensor unit (100) to the applicator (30). A protective sheet opening (162) is formed on one side of the protective sheet (160) to penetrate the protective sheet (160) in the thickness direction. The protective sheet (160) is attached to the adhesive layer (155) such that the boss (127) is inserted into the protective sheet opening (162).

The shape of the protective sheet (160) is not limited to that shown and can be changed in various ways.

An adhesive member (164) is provided on the surface of the protective sheet (160). The adhesive member (164) may be in the form of a double-sided tape with adhesive properties on both sides. One side of the adhesive member (164) may be adhered to the protective sheet (160), and the other side of the adhesive member (164) may be adhered to the removing member (610) provided in the applicator (30). Accordingly, the protective sheet (160) can be coupled to the removing member (610) through the adhesive member (164).

The shape, number, and location of the adhesive members (164) are not limited to those shown and can be changed in various ways.

The sensor unit (100) is mounted on the applicator (30) with the protective sheet (160) attached to the adhesive layer (155). The protective sheet (160) may be separated from the adhesive layer (155) by a removing unit (600) of the applicator (30) before the sensor (110) is inserted into the skin of a user. The sensor unit (100) moves toward the base unit (200) with the protective sheet (160) separated and is coupled to the base unit (200).

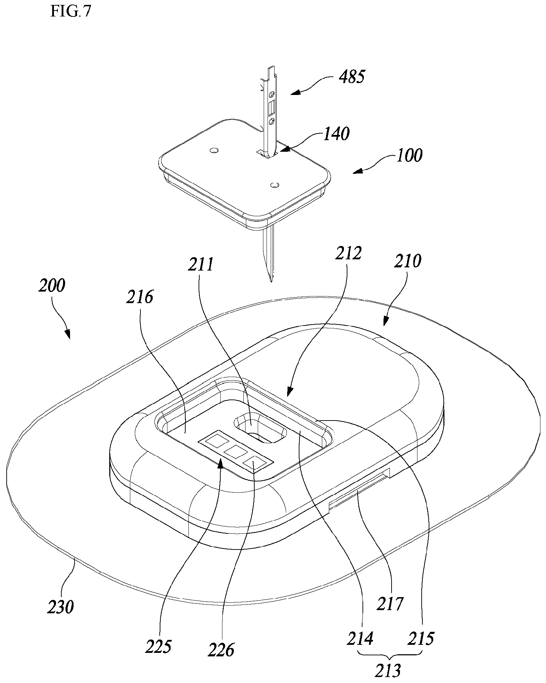

As shown in FIGS. 7 to 9, the base unit (200) includes a base unit housing (210) to which the sensor unit 100 is coupled, and electronic components installed inside the base unit housing (210). The electronic components may include a substrate (223), a base unit electrical contact portion (225) contacting the sensor unit electrical contact portion (146) of the sensor unit (100), a battery (228), and so on. The board (223) may be equipped with a processor chip for processing signals or a communication chip for wireless communication with an external terminal (5).

The base unit housing (210) is provided with an insertion hole (211) through which the insertion portion (116) of the sensor (110) and the needle (485) can pass, and a mounting portion (212) in which the sensor unit housing (120) is coupled. The mounting portion (212) includes a base unit recess (213) and a contact surface (216) provided inside the base unit recess (213). The insertion hole (211) is formed to be shaped such that the boss (127) of the sensor unit (100) can be fitted and is disposed inside the base unit recess (213). The contact surface (216) may be formed to be flat such that the adhesive layer (155) of the sensor unit (100) can be stably adhered. The base unit recess (213) includes a first recess (214) connected to the insertion hole (211), and a second recess (215) that is located farther from the insertion hole (211) than the first recess (214) and is connected to the first recess (214). The second recess (215) is wider than the first recess (214). The first recess (214) has a shape corresponding to the body portion (143) of the sensor unit housing (120), and the second recess (215) is formed to have a shape corresponds to the cover portion (144) of the sensor unit housing (120). Accordingly, the sensor unit housing (120) can be fitted and coupled to the base unit recess (213), thereby maintaining a stable coupling state with the base unit housing (210). In addition, since the sensor unit housing (120) is stably fitted and coupled to the base unit housing (210), moisture or foreign substances cannot easily enter the gap between the sensor unit housing (120) and the base unit housing (210). A housing groove (217) is formed on the outer edge of the base unit housing (210). Some part of a locking hook (360) provided on the applicator (30) may be inserted into the housing groove (217).

A base unit electrical contact (225) is arranged at a mounting portion (212) and contacts a sensor unit electrical contact (146) of the sensor unit (100) when sensor unit (100) is coupled to base unit (200). The base unit electrical contact portion (225) is electrically connected to the substrate (223) and is installed at the base unit housing (210) so that a portion of the base unit electrical contact portion (225) is exposed to the base unit recess (213). The base unit electrical contact portion (225) may include a plurality of terminal portions (226) for transmitting electrical signals. The terminal portion (226) may electrically connect the sensor unit electrical contact portion (146) and the substrate (223) by contacting the terminal portion (147) of the sensor unit (100).

As shown in the drawings, the base unit housing (210) may include a lower housing (219) and an upper housing (221) covering the upper part of the lower housing (219), but their shapes may be changed in various ways.

An adhesive portion (230) is provided on the surface of the base unit housing (210). The adhesive portion (230) is attached to the surface of the lower housing (219) to adhere the base unit housing (210) to the skin of a user. In the middle of the adhesive portion (230), an adhesive hole (231) through which the insertion portion (116) of the sensor (110) and the needle (485) can pass is formed to penetrate the adhesive portion (230) in the thickness direction.

The adhesive portion (230) may be covered and protected with a protective sheet. The protective sheet covering the adhesive portion (230) may be removed during the process of attaching the base unit (200) to the skin of a user.

The sensor unit (100) and the base unit (200) are installed in the applicator (30) in a separated state, and are coupled to each other in the process of operating the applicator (30) to insert the sensor (110) into the skin of a user, forming the body attachable unit (20). The sensor unit (100) may be mounted on the applicator (30) while the needle (485) coupled, and moved toward the base unit (200) with the needle (485) coupled thereto to be coupled to the base unit (200). After the sensor unit (100) is coupled to the base unit (200), the needle (485) is separated from the sensor unit (100), and only the body attachable unit (20) remains on the skin of a user.

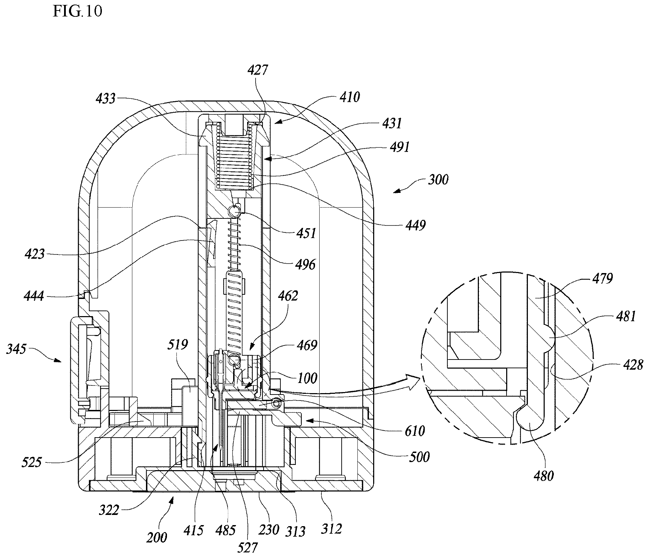

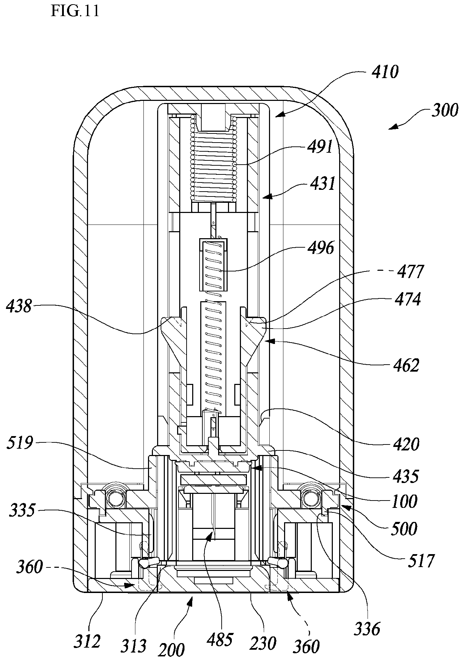

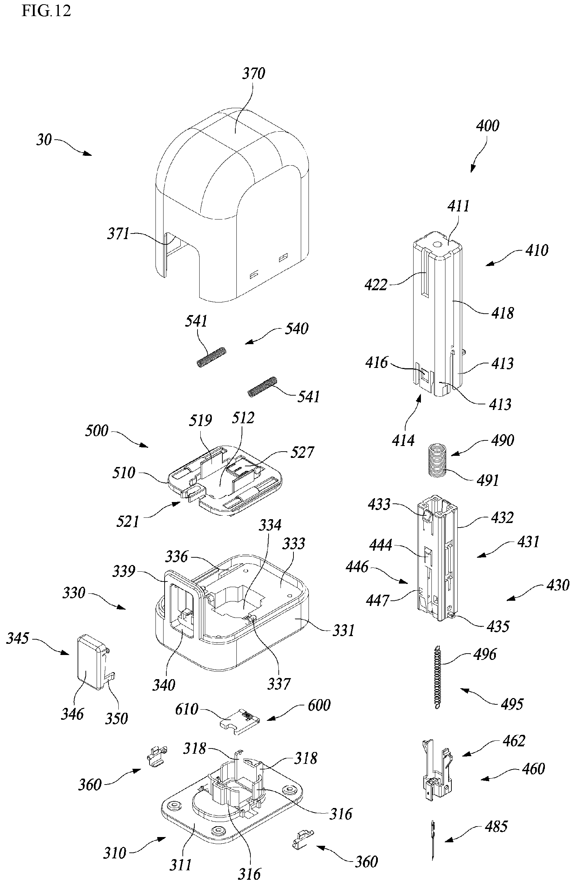

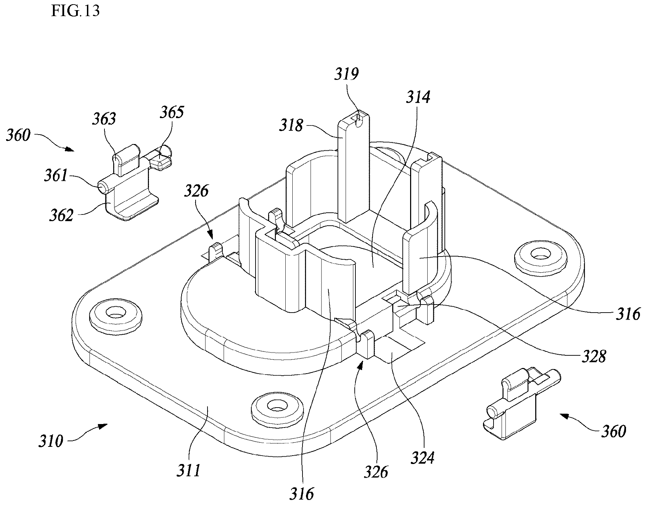

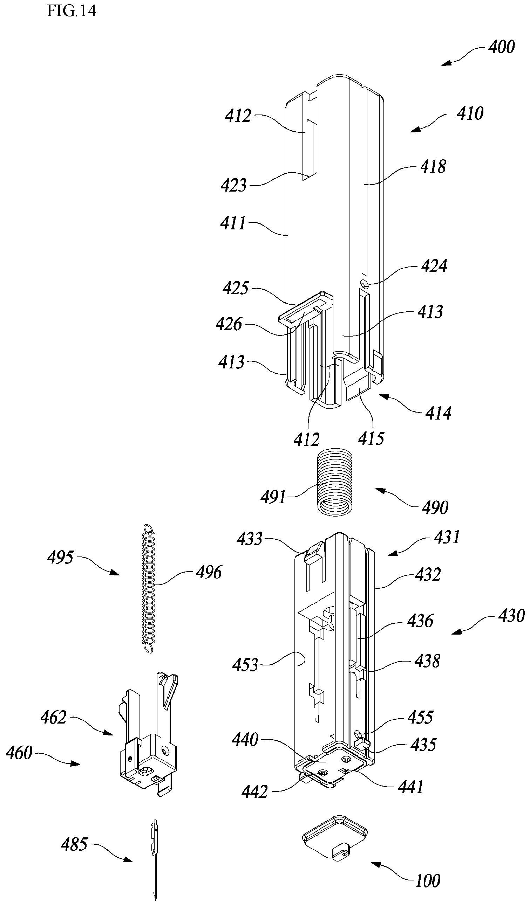

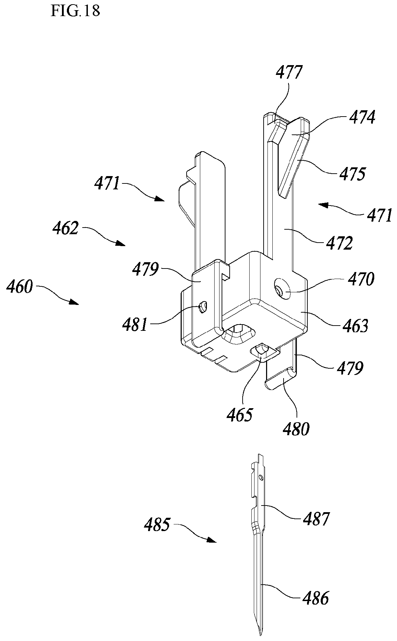

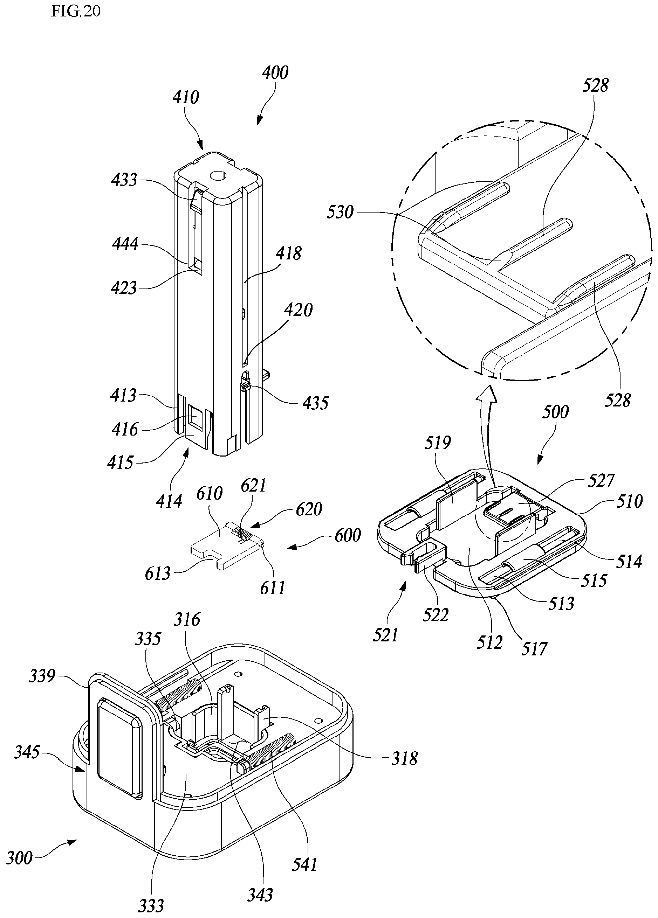

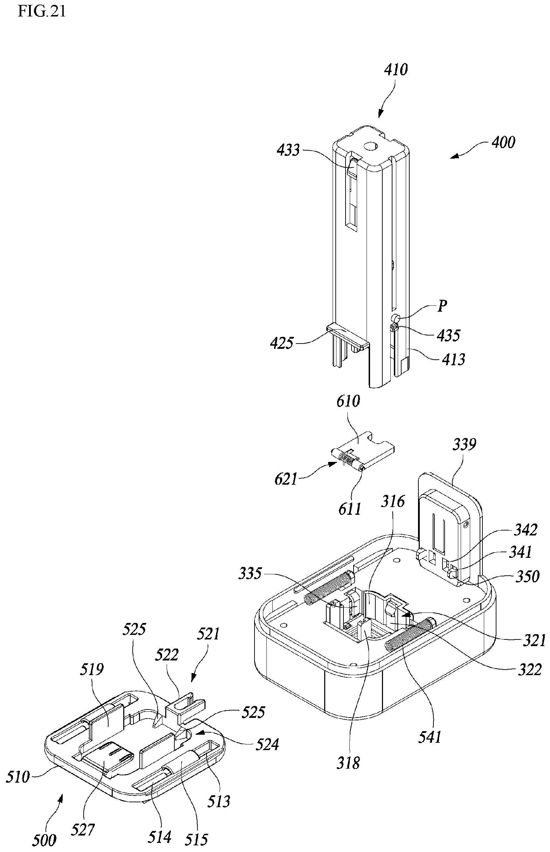

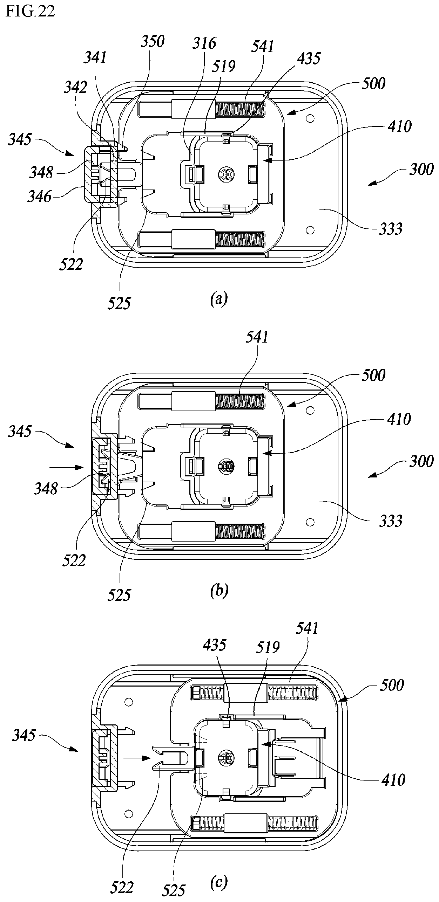

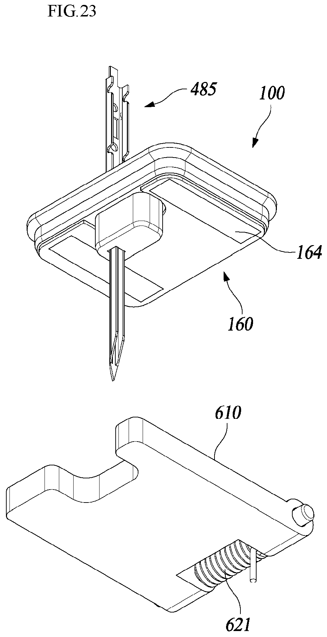

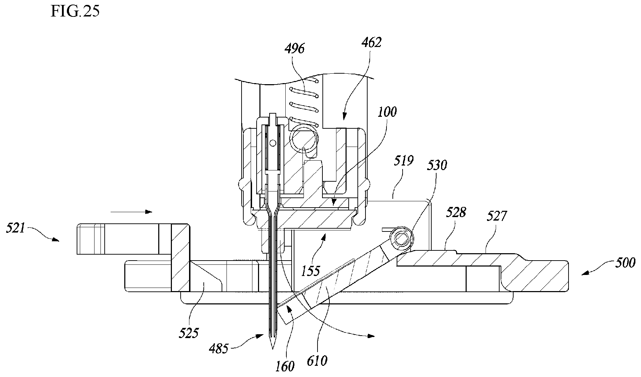

Referring to FIGS. 10 to 25, the applicator (30) operates with the sensor unit (100) and the base unit (200) mounted, thereby coupling the sensor unit (100) and the base unit (200) and can attach the body attachable unit (20) in which the sensor unit (100) and the base unit (200) are coupled to the skin of a user. The sensor unit (100) and the base unit (200) are spaced apart from each other and are respectively mounted on the applicator (30). The applicator (30) is separated from the body attachable unit (20) after attaching the body attachable unit (20) to the skin of a user, and the body attachable unit (20) remains attached to the skin of a user by the adhesive portion (230).

The applicator (30) includes an applicator body (300) to which the base unit (200) is detachably coupled, an insertion unit assembly (400) that includes an insertion unit (430) for inserting the sensor (110) into the skin of a user, a stopper member (500) movably installed on the applicator body (300), and a removing unit (600) for separating the protective sheet (160) of the sensor unit 100. The insertion unit (430) may move the sensor unit (100) from a first position spaced apart from the base unit (200) by a preset distance to a second position coupled with the base unit

(200). The sensor unit (100) and the base unit (200) are coupled at the second position to form the body attachable unit (20).

The applicator body (300) includes a base frame (310), a middle frame (330) disposed on the base frame (310), and a top case (370) that is coupled to the middle frame (330) and covers the upper part of the middle frame (330).

The base frame (310) includes a frame base portion (311) having a bottom portion (312) that can be in contact with the skin of a user. The bottom portion (312) of the frame base portion (311) is provided with a recess (313) into which the base unit (200) is mounted. The base unit (200) is detachably coupled to the recess (313) so that the adhesive portion (230) can face the skin of a user and placed in a second position spaced apart from the sensor unit (100). A base frame opening (314) is formed in the middle of the frame base portion (311) to penetrate the frame base portion (311) in the thickness direction. A fence portion (316) and a support (318) are provided to protrude around the base frame opening (314). When the base frame (310) is coupled to the middle frame (330), the fence portion (316) and the support (318) are inserted into the middle frame opening (334) of the middle frame (330). The fence portion (316) may support the column member (410) in contact with the outer surface of the column member (410) provided in the insertion unit assembly (400). The support (318) supports the removing member (610) of the removing unit (600). An applicator body snap portion (321) is provided inside the fence portion (316) to secure the column member (410). The applicator body snap portion (321) includes a snap hook (322) engaged with the column member (410). On both sides of the base frame opening (314) of the frame base portion (311), through holes (324) are formed to penetrate the frame base portion (311) in the thickness direction. A support portion (326) and a supporting portion (328) are provided at the upper portion of each of the pair of through holes (324).

A locking hook (360) for detachably fixing the base unit (200) is coupled to the support portion (326). The locking hook (360) may engage the base unit (200) mounted in the recess (313) and secure the base unit (200) without being separated from the recess (313). The locking hook (360) may be disengaged from the base unit (200) after the body attachable unit (20) is attached to the skin of a user. The locking hook (360) may be pivotally coupled to the support portion (326). The locking hook (360) includes a pivot portion (361) rotatably coupled to the support portion (326), a hook portion (362) connected to the pivot portion (361), and a rod (363) each protruding from the pivot portion (361), and a lever portion (365). The hook portion (362) may be inserted into the housing groove (217) of the base unit (200) to be engaged with the base unit (200). The hook portion (362) protrudes from one side of the pivot portion (361), and the rod (363) protrudes from the other side of the pivot portion (361). The hook portion (362) is inserted into the through hole (324) of the base frame (310), and a portion thereof is located in the recess (313) of the base frame (310). The lever portion (365) protrudes from the pivot portion (361) toward the base frame opening (314). The locking hook (360) can be disengaged from the base unit (200) by rotating about the pivot portion (361) when the lever portion (365) is pressed by the shuttle (431) of the insertion unit assembly (400). When the locking hook (360) rotates and is disengaged from the base unit (200), the lever portion (365) comes into contact with the support portion (328) of the base frame (310). As the lever portion (365) contacts the support portion (328), the rotation angle of the locking hook (360) may be limited and the moving distance of the shuttle (431) may be limited. A more specific operation of the locking hook (360) will be described later.

In addition, the specific configuration of the base frame (310) is not limited to be shown and can be changed in various ways.

The middle frame (330) includes an outer frame (331) coupled to the base frame (310), a stage (333) disposed inside the outer frame (331), and a support fixture (339) that protrudes higher than the stage (333). The stage (333) is disposed on the frame base portion (311). **In** the middle of the stage (333), a middle frame opening (334) into which the fence portion (316) and the support (318) of the base frame (310) are inserted is provided. The middle frame opening (334) is formed to correspond to the base frame opening (314) of the base frame (310). That is, when the base frame (310) and the middle frame (330) are coupled, the middle frame opening (334) and the base frame opening (314) are connected. The middle frame opening (334) is connected to the base frame opening (314) to form an applicator body groove (343). A portion of the insertion unit assembly (400) may be inserted into the applicator body groove (343), and the insertion unit assembly (400) may be coupled with the applicator body (300). Both sides of the middle frame opening (334) are provided with an elastic arm (335) supporting the locking hook (360).

The elastic arm (335) may contact the rod (363) of the locking hook (360) to apply an elastic force to the locking hook (360) in a direction of being engaged with the base unit (200). That is, the elastic arm (335) may support the locking hook (360) so that the locking hook (360) can maintain a state of being engaged with the base unit (200). **In** the drawings, the elastic arm (335) is located in the middle frame opening (334) so that an elastic force can be applied to the rod (363) in a direction away from the middle frame opening (334), but the installation location of the elastic arm (335) or the position where the elastic arm (335) contacts the locking hook (360) may be variously changed.

The stage (333) is provided with a guide rail (336) and a fixing protrusion (337). The guide rail (336) plays a role in guiding the stopper member (500) linearly. The fixing protrusion (337) supports the stopper member driver (540), which provides mobility to the stopper member (500).

The support fixture (339) supports the operation member (345) for operating the stopper member (500). The support fixture (339) is provided with a support fixture groove (340) for the installation of the operation member (345) and a plurality of support fixture openings (341 and 342).

The operation member (345) includes a button portion (346), a pressing protrusion (348) protruding from the button portion (346), and an operation member hook (350). The operation member (345) is operated by the user to operate the stopper member (500). The operation member (345) may move in the support fixture groove (340) without separating from the support fixture (339) by inserting the operation member hook (350) into the support fixture opening (341) of the support fixture (339). When the operation member (345) is pressed toward the inside of the applicator body (300) by the user, the pressing protrusion (348) may press the fixing hook (522) of the stopper member (500). The stopper member (500) may be fixed to the applicator body (300) by a fixing hook (522), and then, when the operation member (345) presses the fixing hook (522), the fixing force on the applicator body (300) is released and it can move.

In addition to the illustrated configuration, the operation member (345) may be changed to various other configurations installed on the applicator body (300) to be operated by a user to move the stopper member (500).

The top case (370) is coupled with the middle frame (330) and covers the upper part of the middle frame (330). On one side of the top case (370), a top case opening (371) is provided in a shape which allows the support fixture (339) of the middle frame (330) to be coupled.

The specific configuration of the top case (370) is not limited to that shown and can be changed in various ways. Additionally, the method of coupling the top case (370) and the middle frame (330) can also be changed in various ways.

In addition to the configuration including the base frame (310), the middle frame (330), and the top case (370) as shown, the applicator body (300) may be changed to various other configurations that can support the insertion unit assembly (400) by being coupled with the insertion unit assembly (400). Additionally, the applicator body (300) may be coupled to the base unit (200) in various other ways other than using a locking hook.

The insertion unit assembly (400) includes a column member (410) fixed to the applicator body (300) and an insertion unit (430) that is supported on the column member (410) to be coupled with the sensor unit (100) and moves the sensor unit (100) from the first position to the second position.

The column member (410) is fixed to the applicator body (300) and supports the insertion unit (430). The column member (410) includes a column member body (411). The column member body (411) has a space inside to accommodate the insertion unit (430), and has a column member opening (412) opened to the outside at one end. A plurality of column member legs (413) are provided at one end portion of the column member body (411) in a shape surrounding the column member opening (412). The column member leg (413) may be provided in an elastically deformable form. The column member (410) may be coupled to the applicator body (300) in a manner in which the column member leg (413) is inserted into the applicator body groove (343) of the applicator body (300).

Additionally, one end of the column member body (411) is provided with a column member snap portion (414) that can engage with the applicator body snap portion (321) of the applicator body (300). The column member (410) can be more firmly fixed to the applicator body (300) by engaging the column member snap portion (414) with the applicator body snap portion (321). The column member snap portion (414) includes an elastically deformable snap arm (415). The snap arm (415) is provided with a snap groove (416) into which the snap hook (322) of the applicator body snap portion (321) engages. When the column member (410) is inserted into the applicator body groove (343), the snap hook (322) and the snap arm (415) come into contact with each other, and a portion of the snap hook (322) is inserted into the snap groove (416). In this way, the column member (410) can be stably fixed to the applicator body (300) by engaging the snap hook (322) and the snap arm (415). Additionally, the fence portion (316) of the applicator body (300) is in close contact with the outer surface of the column member (410), so that the column member (410) can be supported more stably. In this way, the column member (410) can be simply coupled to the applicator body (300) by inserting one end into the applicator body groove (343) of the applicator body (300).

The method of coupling the applicator body (300) and the column member (410) is not limited to that shown and may be changed in various ways. As another exemplary embodiment, the position or number of applicator body snap portion (321) provided in the applicator body (300) and the position or number of column member snap portion (414) provided in the column member (410) can be changed in various ways. Additionally, a snap groove may be provided at the applicator body snap portion (321), and a snap hook inserted into the snap groove may be provided at the column member snap portion (414). As another exemplary embodiment, the column member (410) may be coupled to the applicator body (300) by forced press-fitting into the applicator body groove (343). As another exemplary embodiment, the column member (410) may be coupled to the applicator body (300) in such a way that a portion of the applicator body (300) is inserted into the column member opening (412) of the column member (410).

Slits (418) are formed on both sides of the column member body (411) in a direction parallel to the moving direction of the sensor unit (100). A release portion (420) is provided in the middle portion of the slit (418). The release portion (420) is a part where a portion of the carrier (462) may contact while the carrier (462) of the insertion unit (430) moves toward the base unit (200). The carrier (462) may be disengaged from the shuttle (431) of the insertion unit (430) by the action of the release portion (420).

Additionally, guide grooves (422) are formed on the other both sides of the column member body (411) in a direction parallel to the moving direction of the sensor unit (100). The shuttle hook (433) of the shuttle (431) is disposed in the guide groove (422), so that the shuttle hook (433) can move along the guide groove (422). A column member detent (423) is provided at the end of the guide groove (422) to limit the moving distance of the shuttle (431). When the shuttle (431) moves from the first position with the sensor unit (100) and reaches the second position, the shuttle hook (433) comes into contact with the column member detent (423) so that the shuttle (431) may stop at the second position. A column member hole (424) is formed on one side of the column member body (411) to penetrate through the side of the column member body (411) from inside to outside. A safety pin (P) may be inserted into the column member hole (424) to restrict the movement of the shuttle (431) and the carrier (462). A cover portion (425) is provided on another side of the column member body (411). The cover portion (425) is disposed to face the support (318) of the applicator body (300) when the column member (410) is coupled to the applicator body (300) and can cover a part of the removing member (610) supported on the support (318). The cover portion (425) is provided with a cover portion groove (426) into which the removing member 610 can be partially inserted. A retention portion (427) is provided inside the column member (410). The retention unit (427) serves to support the shuttle driver (490) which provides a moving force to the shuttle (431). The insertion unit (430) is coupled to the applicator body (300) through the column member (410) and can move the sensor unit (100) from the first position to the second position. The insertion unit (430) includes a shuttle (431) movably installed inside the column member (410) and a needle assembly (460) having a needle (485) that can be moved with the shuttle (431) and inserted into the skin of a user.

The shuttle (431) is installed to be linearly movable inside the column member (410). In a state in which the column member (410) is coupled to the applicator body (300), the shuttle (431) can move from the first position to the second position with the sensor unit (100). The shuttle (431) includes a shuttle body (432) with a space inside to accommodate the needle assembly (460). A shuttle hook (433) is provided on one side of the shuttle body (432) to limit the movement range of the shuttle (431). The shuttle hook (433) protrudes from the shuttle body (432) and is inserted into the guide groove (422) of the column member (410), and when the shuttle (431) moves to the second position, the movement of the shuttle (431) may be restricted by contacting the column member detent (423) of the column member (410). A shuttle protrusion (435) is provided on the other side of the shuttle (431). The shuttle protrusion (435) protrudes from the shuttle body (432) so as to contact the stopper member (500). The shuttle (431) is fixed in the first position when the shuttle protrusion (435) comes into contact with the stopper member (500), and can move to the second position when the shuttle protrusion (435) leaves the stopper member (500). The shuttle (431) can move the locking hook (360) by the shuttle protrusion (435). That is, when the shuttle (431) reaches the second position, the shuttle protrusion (435) presses the lever portion (365) of the locking hook (360), so that the locking hook (360) may rotates and be disengaged from the base unit (200).

A pair of slits (436) are formed on both sides of the shuttle body (432) in a direction parallel to the moving direction of the shuttle (431). The middle portion of the slit (436) is provided with a shuttle detent (438) with which the carrier latch (477) of the carrier (462) can be engaged. In a state in which the carrier latch (477) engaged with the shuttle detent (438), the carrier (462) moves with the shuttle (431) and cannot relatively move with respect to the shuttle (431). And when the carrier latch (477) is disengaged from the shuttle detent (438), the carrier (462) can relatively move with respect to the shuttle (431).

A shuttle bottom portion (440) in contact with the sensor unit (100) is provided at one end of the shuttle body (432). The shuttle bottom portion (440) is provided with a shuttle through hole (441) and a pressing protrusion (442). A needle (485) which is movable inside the shuttle body (432) may protrude to the outside of the shuttle body (432) through the shuttle through hole (441). The pressing protrusion (442) is provided to be inserted into the sensor unit groove (138) of the sensor unit (100). When the sensor unit (100) is mounted on the insertion unit (430), the pressing protrusion (442) is inserted into the sensor unit groove (138) of the sensor unit (100), so that the sensor unit (100) can maintain its fixed position without moving.

A stopper arm (444) which is elastically deformable is provided on the side of the shuttle body (432). The stopper arm (444) may be used to temporarily fix the shuttle (431) to the column member (410) during the process of assembling the insertion unit assembly (400). The insertion unit assembly (400) may be coupled to the applicator body (300) in a state in which the shuttle (431) is temporarily fixed to the column member (410). While the shuttle (431) is temporarily fixed to the column member (410) by the stopper arm (444), the shuttle (431) cannot move. When the insertion unit assembly (400) is coupled to the applicator body (300) in a state that the shuttle (431) is temporarily fixed to the column member (410), the insertion unit (430) does not operate even if the user presses the operating member (345). Therefore, in order for the applicator 30 to be in an operable state, it is necessary to couple the insertion unit assembly (400) to the applicator body (300) and release the temporarily fixed state of the shuttle (431). When the shuttle (431) is inserted into the inside of the column member (410) and the stopper arm (444) is engaged with the column detent (423) of the column member (410) In the process of assembling the insertion unit assembly (400), the shuttle (431) is temporarily fixed to the column member (410). At this time, since the stopper arm (444) is engaged with the column member detent (423), the shuttle (431) cannot move.

There are various advantages to temporarily fixing the shuttle (431) to the column member (410) during the process of assembling the insertion unit assembly (400). That is, if the shuttle (431) is temporarily fixed to the column member (410), accidental movement of the shuttle 431 can be prevented in the process of assembling the insertion unit assembly (400) or in the process of coupling the insertion unit assembly (400) to the applicator body (300). Additionally, since a sharp needle (485) is coupled to the shuttle (431), the risk of a safety accident due to accidental movement of the needle (485) during the manufacturing process can be reduced.

The drawings show that the shuttle (431) is temporarily fixed to the column member (410) in a way that the stopper arm (444) included in the shuttle (431) is engaged with the column member detent (423) of the column member (410), but the temporary fixing type of the shuttle (431) may be changed in various ways. As another exemplary embodiment, the stopper arm (444) may be included in the column member (410) to be engaged with the shuttle (431). Additionally, it is possible to temporarily fix the shuttle (431) to the column member (410) using various other types of components in addition to the elastically deformable stopper arm.

A shuttle locking portion (446) is provided on one side of the shuttle body (432). The shuttle locking unit (446) is for fixing the shuttle 431, which has reached the second position, at the second position. After the sensor (110) is inserted into the user's skin by the operation of the applicator (30), the applicator (30) needs to be separated from the body attachment unit (20) and discarded. Because the needle (485) of the applicator (30) is inserted into the user's skin in the process of inserting the sensor (110) into the user's skin and then extracted from the user's skin, the needle (485) is contaminated after the use of the applicator (30). Additionally, when the shuttle (431) is moved to the first position and reloaded after the use of the applicator (30), the needle (485) may move accidentally, thereby leading to a safety accident. To prevent this problem, the shuttle (431) is moved to the second position and then fixed to the second position by the shuttle locking unit (446). After the use of the applicator (30), the applicator (30) cannot be reused because the shuttle (431) is fixed to the second position. The shuttle locking portion (446) includes a locking groove (447) into which the locking protrusion (525) of the stopper member (500) can be inserted.

A retention portion (449) and a fixing portion (451) are provided inside the shuttle (431). The retention portion (449) supports the shuttle driver (490). A needle release driver (495) that provides a moving force to the carrier (462) is coupled to the fixing portion (451).

In addition, the shuttle (431) includes a shuttle opening (453) and a shuttle hole (455). The shuttle opening (453) is formed at one side portion of the shuttle body (432) to be open outward. In the process of assembling the insertion unit assembly (400), the needle assembly (460) may be arranged inside the shuttle body (432) through the shuttle opening (453). The shuttle hole (455) is formed to penetrate the side portion of the shuttle (431) inside and out. A safety pin (P) may be inserted into the shuttle hole (455). When the safety pin (P) passes through the column member hole (424) and is inserted into the shuttle hole (455), the movement of the shuttle (431) may be restricted. The shuttle 431 can be temporarily fixed to the column member (410) by inserting the safety pin (P) into the shuttle hole (455).

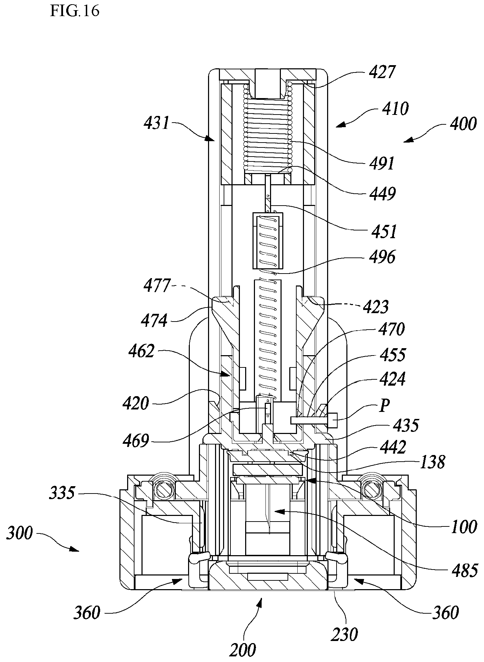

The shuttle (431) moves by receiving movement force from the shuttle driver (490). The shuttle driver (490) includes an elastic member (491) that applies elastic force to the shuttle (431) in a direction in which it moves from the first position to the second position. The elastic member (491) has one end in contact with the retention portion (427) of the column member (410) and the other end in contact with the retention portion (449) of the shuttle (431). The shuttle (431) may maintain a state in which the elastic member (491) is elastically deformed and temporarily fixed to the column member (410) by the stopper arm (444) and the safety pin (P). After the insertion unit assembly (400) is coupled to the applicator body (300), the stopper arm (444) is disengaged from the column member detent (423) of the column member (410), and when the safety pin (P) is separated from the shuttle (431), the shuttle protrusion (435) comes into contact with the stopper member (500), and the shuttle (431) is converted to a movable state by the operation member (345). As shown in (a) of FIG. 16, when the stopper member (500) comes into contact with the shuttle protrusion (435) and supports the shuttle protrusion (435), the shuttle (431) may be fixed in the first position. And, as shown in (c) of FIG. 16, when the stopper member (500) moves and deviates from the shuttle protrusion (435), the shuttle (431) moves to the second position by the elastic force of the elastic member (491).

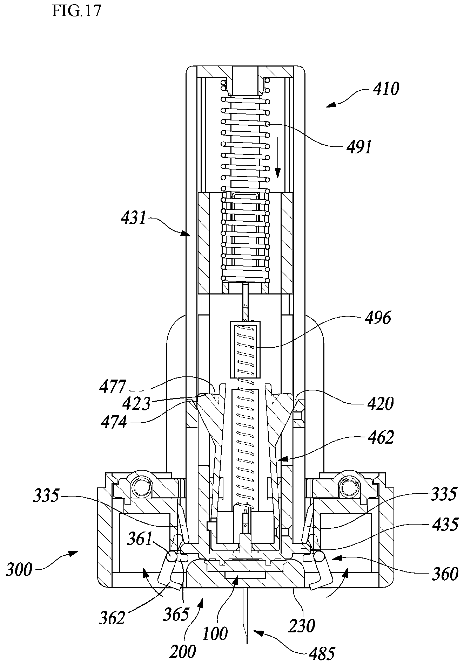

The shuttle (431) moves the sensor unit (100) toward the base unit (200) by moving from the first position to the second position and reaches the second position to release the locking hook (360) fixing the base unit (200) to the applicator body (300). As shown in FIG. 17, when the shuttle (431) reaches the second position, the shuttle protrusion (435) comes into contact with the lever portion (365) of the locking hook (360) and presses the lever portion (365). At this time, the locking hook 360 rotates the elastic arm (335) around the pivot portion (361) while biasing the elastic arm (335) of the applicator body (300), thereby causing the hook portion (362) to be out of the base unit housing (210). Accordingly, the base unit (200) may be disengaged from the locking hook (360) and separated from the applicator body (300).

The shuttle driver (490) may be changed to various other configurations that can provide a moving force to the shuttle (431) in addition to the configuration including the elastic member (491) in the form of a coil spring.

The needle assembly (460) includes a carrier (462) to which the sensor unit (100) is detachably coupled, and a needle (485) coupled to the carrier (462) to penetrate the sensor unit (100). The needle (485) may be coupled to the sensor unit (100) and move forward from the first position to the second position with the sensor unit (100) to be inserted into the skin of a user, and may be pulled back together with the carrier (462) at the second position to be separated from the skin of a user and the sensor unit (100).

The carrier (462) is coupled to the shuttle (431) to be relatively movable. A portion of the carrier (462) engages with the shuttle (431) so that it can advance together with the shuttle (431) from the first position to the second position. Then, the carrier (462) is disengaged from the shuttle (431) in the second position and may retreat in a direction away from the sensor unit (100).

The carrier (462) includes a carrier body (463) to which the needle (485) is coupled, an elastically deformable carrier wing (471) extending from the carrier body (463), and a grip arm (479) connected to the side of the carrier body (463).

The carrier body (463) includes a boss (464) with an insertion groove (465) formed therein. A portion of the needle (485) may be inserted into the insertion groove (465). A clamp portion (467) configured to engage with the needle 485 to fix the needle (485) is provided in the insertion groove (465). A fixing portion (469) to which the needle release driver (495) is coupled is provided at one side of the carrier body (463). The carrier (462) can be movable relative to the shuttle (431) by the needle release driver (495).

The needle release driver (495) provides a moving force to the carrier (462). The needle release driver (495) includes an elastic member (496) whose one end is coupled to the fixing portion (451) of the shuttle (431) and the other end is coupled to the fixing portion (469) of the carrier (462). The elastic member (496) is in the form of a tension spring and applies an elastic force to pull the carrier (462) toward the fixing portion (451) of the shuttle (431). That is, the elastic member (496) can apply an elastic force to the carrier (462) in a direction away from the sensor unit (100) to move the needle (485) coupled to the carrier (462) to be separated from the sensor unit (100).

The needle release driver (495) can be changed to various other configurations that can provide a moving force to the carrier (462) in addition to the configuration including the elastic member (496) in the form of a coil spring.

A carrier hole (470) is formed on the side of the carrier body (463) to penetrate the side of the carrier body (463) from inside to out. A safety pin (P) may be inserted into the carrier hole (470). The safety pin (P) sequentially passes through the column member hole (424) of the column member (410) and the shuttle hole (455) of the shuttle (431) and then is inserted into the carrier hole (470) thereby temporarily fixing the carrier (462) to the shuttle (431).

In the process of assembling the insertion unit assembly (400) by temporarily fixing the shuttle (431) and the carrier (462) using a safety pin (P), or in the process of coupling the insertion unit assembly (400) to the applicator body (300), a problem in which the shuttle (431) and the carrier (462) are accidentally moved may be reduced.

As shown in FIG. 16, the safety pin (P) can be inserted into the column member hole (424) of the column member (410), the shuttle hole (455) of the shuttle (431), and the carrier hole (470) of the carrier (462). In a state that the column member hole (424), the shuttle hole (455), and the carrier hole (470) are aligned in a line, the safety pin (P) sequentially passes through the column member hole (424) and the shuttle hole (455) and is inserted to the carrier hole (470). At this time, the shuttle (431) and the carrier (462) cannot move. Therefore, the safety pin (P) can prevent accidental movement of the shuttle (431) and the carrier (462) during the process of assembling the insertion unit assembly (400) or the process of coupling the insertion unit assembly (400) to the applicator body (300). When the safety pin (P) is separated from the carrier (462), the shuttle (431), and the column member (410), the insertion unit (430) becomes operable by the operation member (345).

The specific configuration and number of safety pins (P) can be changed in various ways. As another exemplary embodiment, a safety pin passing through the column member may be installed to be engaged with the shuttle without reaching the carrier. In this case, the safety pin can restrict the movement of the shuttle at the first position to prevent malfunction of the applicator due to the user's carelessness.

Additionally, the use of a safety pin (P) may be excluded during the manufacturing process of the applicator (30). In this case, the shuttle (431) can be temporarily fixed to the column member (410) using the stopper arm (444).

The carrier wing (471) is connected to the carrier body (463) in a shape that extends from the carrier body (463) in the moving direction of the carrier (462). The carrier wing (471) includes a wing body (472) elastically deformably connected to the carrier body (463), and a trigger (474) and a latch portion (477) protruding from the wing body (472).

The trigger (474) is provided on one side of the wing body (472) to protrude outward from the wing body (472). The trigger (474) is inserted into the slit (436) of the shuttle (431) and the slit (418) of the column portion (410) and can move along these slits (436 and 418). The trigger (474) is inserted into the slits (436)(418) and guided by the slits (436)(418) so that the carrier (462) can move linearly more stably. While the carrier (462) moves from the first position to the second position together with the shuttle (431), the trigger (474) may contact the release portion (420) disposed in the middle of the slit (418) of the column member (410).

As shown in FIG. 17, the release portion (420) is disposed in the middle of the slit (418) of the column member (410) to press the trigger (474). While the carrier (462) is moving from the first position to the second position, the trigger (474) may come into contact with the release portion (420) and the carrier wing (471) may be elastically deformed. The trigger (474) is provided with a trigger inclined portion (475) that contacts the release portion (420). While the carrier (462) is moving from the first position to the second position, the trigger inclined portion (475) comes into contact with the release portion (420) so that the carrier wing (471) can be elastically deformed more smoothly, and the impact that occurs when the trigger (474) contacts the release portion (420) can be reduced.

The carrier latch (477) protrudes outward from the wing body (472). The direction in which the carrier latch (477) protrudes from the wing body (472) is the same as the direction in which the trigger (474) protrudes from the wing body (472). The protrusion height at which the carrier latch (477) protrudes from the wing body (472) is lower than the protrusion height at which the trigger (474) protrudes from the wing body (472). The carrier latch (477) may be engaged with a shuttle detent (438) of the shuttle (431). In a state that the carrier latch (477) is engaged with the shuttle detent (438), the carrier (462) can maintain a state that the elastic member (496) is elastically deformed and cannot move in the direction to which the elastic force of the elastic member (496) is applied. And, when the trigger (474) contacts the release portion (420) and the carrier wing (471) is elastically deformed, the carrier latch (477) may deviate from the shuttle detent (438) and therefore the carrier (462) may move.

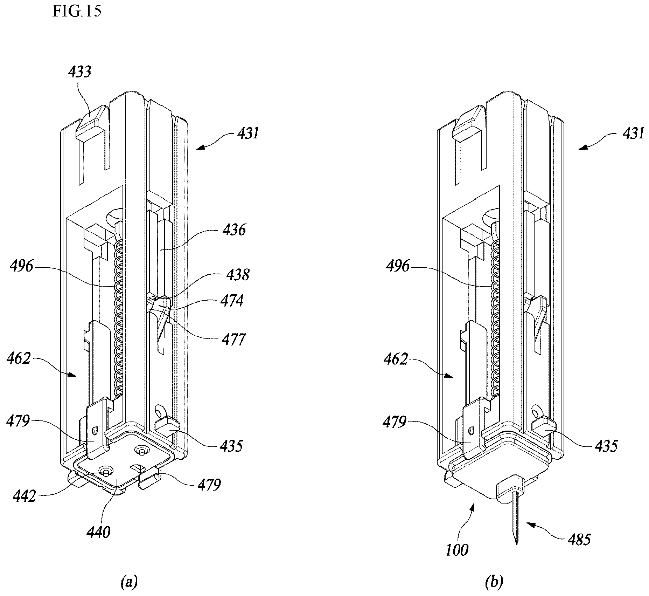

The grip arm (479) is provided on the side of the carrier body (463) to be elastically deformable. A pair of grip arms (479) are arranged to face each other on both sides of the carrier body (463) and detachably fix the sensor unit housing (120) of the sensor unit (100). The grip arm (479) is provided with a grip protrusion (480) engaged with the sensor unit housing (120) and a protrusion (481) in contact with the inner wall (428) of the column member (410). As illustrated in FIG. 15(a), the carrier (462) is coupled to the shuttle (431) so that the carrier body (463) is located inside the shuttle (431) and the end of the grip arm (479) protrudes from the shuttle (431). As illustrated in FIG. 15(b), the carrier (462) may fix the sensor unit (100) in such a way that a pair of grip arms (479) engage with the sensor unit housing (120). At this time, the surface of the sensor unit housing (120) is in close contact with the shuttle bottom portion (440) of the shuttle (431), and the pressing protrusion (442) of the shuttle (431) is inserted into the sensor unit groove (138) of the sensor unit housing (120). Therefore, the sensor unit (100) can remain stably coupled to the shuttle (431).

And the carrier (462) may position the sensor unit (100) in the first position when the insertion unit assembly (400) is coupled to the applicator body (300), as shown in FIG. 10. When the carrier (462) is located inside the column member (410), the protrusion (481) of the grip arm (479) comes in contact with the inner wall (428) of the column member (410).

Meanwhile, when the sensor unit (100) and the carrier (462) move toward the base unit (200) and the sensor unit (100) is coupled to the base unit (200), the protrusion portion (481) is located at a space connected with a recess (131), in which the base unit (200) is accommodated, to deviate from the inner wall (428) of the column member (410). Therefore, in a state that the sensor unit (100) is coupled to the base unit (200), the fixing force of the grip arm (479) is weakened. Because the sensor unit (100) is fixed to the base unit (200) by the adhesive layer (155), when the carrier (462) is pulled in a direction of being away from the body attachable unit (20) by the needle release driver (495), the grip arm (479) can be smoothly disengaged from the sensor unit (100) and separated from the sensor unit (100).

In addition to the configuration shown, the carrier (462) can be changed to various other configurations in which it is coupled to the needle (485) and installed to be relatively movable with respect to the shuttle (431). For example, the drawings show that a pair of carrier wings (471) are symmetrically provided on both sides of the carrier body (463), but the number and shape of the carrier wings (471) can be changed in various ways. Additionally, the number and shape of the grip arms (479) may be changed in various ways. In addition, the way of coupling the carrier (462) and the needle (485) and the way of coupling the carrier (462) and the sensor unit (100) may also be changed in various ways.

The needle (485) can move from the first position to the second position in a state that the needle (485) is fixed to the carrier (462) and coupled to the sensor unit (100). The needle (485) is formed to have a sharp tip such that the needle (485) can pierce the skin of the user and be smoothly inserted into the user's skin. When the sensor unit (100) moves to the second position, the needle (485) penetrates the user's skin before the sensor 110 to allow the sensor (110) to be stably inserted into the skin. The needle (485) is separated from the user's skin after the sensor (110) is inserted into the user's skin. The needle (485) includes a needle body (486) that can be inserted into the user's skin, and a needle head (487) coupled to the carrier (462). The needle (485) can maintain a state of being stably coupled to the carrier (462) by inserting the needle head (487) into the inside of the insertion groove (465) of the carrier (462) and being engaged with the clamp portion (467).

The specific configuration of the needle (485) is not limited to that shown and may be changed in various ways. Additionally, the method in which the needle (485) is coupled to the carrier (462) may also vary.

The insertion unit assembly (400) may be coupled to the applicator body (300) in a state in which the elastic member (491) of the shuttle driver (490) which is for moving the shuttle (431) and the elastic member (496) of the needle release driver (495) which is for moving the needle assembly (460) are elastically deformed. Additionally, the insertion unit assembly (400) may be coupled to the applicator body (300) while being coupled to the sensor unit (100).

As described above, in the process that the insertion unit assembly (400) is coupled to the applicator body (300), the shuttle (431) is temporarily fixed to the column member (410) by the stopper arm (444) and the safety pin (P), and the carrier (462) can be temporarily fixed to the shuttle (431) by the safety pin (P), and therefore accidental movement of the shuttle (431) and the carrier (462) can be prevented. After the insertion unit assembly (400) is coupled to the applicator body (300), the safety pin (P) is removed and the stopper arm (444) is manipulated to be released from the restraining force on the shuttle (431), so that the insertion unit (430) becomes a state of being operable by the operation member (345). The stopper arm (444) can be disengaged from the column member detent (423) of the column member (410) in a simple manner by being pressed toward the inside of the column member (410) using a simple tool. For example, when the user presses the stopper arm (444) toward the inside of the column member (410), the stopper arm (444) moves away from the column member detent (423) of the column member (410) and the shuttle (431) is slightly pushed by the elastic member (491) of the driver (490), such that a state that the stopper arm (444) is out of the column member detent (423) can be maintained.

As such, the applicator assembly (10) according to an embodiment of the present invention includes a shuttle (431) constituting the insertion unit (430), a needle assembly (460), a shuttle driver (490), and a needle release driver (495) which form one assembly together with the column member (410) and can be simply coupled to the applicator body (300). Therefore, the installation of the insertion unit (430) is easy, the task of installing the insertion unit (430) to the applicator body (300) can be automated, and the manufacturing time can be reduced. Additionally, the sensor unit (100) can be simply installed to the applicator body (300) in a state that the sensor unit (100) is coupled to the insertion unit (430) from the outside of the applicator body (300).

Additionally, the applicator assembly (10) according to an embodiment of the present invention may be coupled to the applicator body (300) in a locked state to prevent accidental operation. And, after the insertion unit (430) is coupled to the applicator body (300), it can be converted to an operable state through simple manipulation. Therefore, after installing the insertion unit (430) to the applicator body (300), there is no need for cumbersome follow-up work to convert the shuttle (431) and carrier (462) of the insertion unit (430) into an operable state.

As another exemplary embodiment, the insertion unit (430) is coupled to the applicator body (300) through the column member (410), and then the shuttle (431) and the carrier (462) can be moved to an operable position by the user.

The stopper member (500) is arranged to move linearly on the stage (333) of the middle frame (330). The stopper member (500) is installed to move in a direction intersecting the direction in which the sensor unit (100) moves from the first position to the second position. In the drawing, the stopper member (500) is shown to move in a direction perpendicular to the direction of movement of the sensor unit (100), but the movement direction of the stopper member (500) may be changed in various ways. The stopper member (500) may move from a third position which supports the shuttle (431) to be positioned in the first position to a fourth position where it disengages from the shuttle (431). In the third position, the stopper member (500) restricts the movement of the shuttle (431) placed in the first position by contacting the shuttle protrusion (435) of the shuttle (431) located in the first position, and when the stopper member (500) moves to the fourth position, it can deviate from the shuttle protrusion (435) so that the shuttle (431) can move to the second position. Additionally, the stopper member (500) may restrict the movement of the removing member (610) in the third position so that the removing member (610) of the removing unit (600) maintains a position coupled to the adhesive layer (155) of the sensor unit (100) located in the first position.

The stopper member (500) can move by receiving a moving force from the stopper member driver (540). The stopper member driver (540) provides a moving force to the stopper member (500) to move the stopper member (500) from the third position to the fourth position. The stopper member driver (540) includes an elastic member (541) that applies elastic force in a direction that deflects the stopper member (500) toward the fourth position. The elastic member (541) may have one end coupled to the fixing protrusion (337) of the middle frame (330) and the other end in contact with the stopper member (500) to apply an elastic force to the stopper member (500).

The stopper member driver (540) may be changed into various other forms that can provide a moving force to the stopper member (500) in addition to the configuration including the elastic member (541) in the form of a coil spring.

The stopper member (500) includes a stopper member body (510), a supporting portion (519) for supporting the shuttle (431), a stopper member fixing portion (521) capable of engaging with the applicator body (300), a stopper member locking portion (524) for fixing the shuttle (431), which has reached the second position, to the second position, and a stopper portion (527) capable of supporting the removing member (610).

A stopper member opening (512) and a stopper member groove (513) are formed in the stopper member body (510) to penetrate the stopper member body (510) in the thickness direction. The stopper member opening (512) is formed at the middle of the stopper member body (510), and a pair of stopper member grooves (513) are arranged on both sides of the stopper member opening (512). A fixing protrusion (514) to which the other end of the elastic member (541) is coupled is provided in the stopper member groove (513). A cover portion (515) is provided above the stopper member groove (513) to cover the elastic member (541) placed in the stopper member groove (513).

A rail portion (517) is provided on one surface of the stopper member body (510) facing the stage (333) of the middle frame (330). The rail portion (517) may engage with the guide rail (336) of the middle frame (330) and slide along the guide rail (336). The drawings show that the guide rail (336) is formed to have a groove shape and the rail portion (517) is inserted into the guide rail (336), but the guide rail (336) and the rail portion (517) are can be changed to a variety of different shapes that can guide the stopper member body (510) to move stably and linearly on stage (333).

A pair of supporting portions (519) are disposed at both edges of the stopper member opening (512) to face each other. The supporting portion (519) may protrude from the stopper member body (510) and support the shuttle protrusion (435) of the shuttle (431). The length of the support portion (519) is shorter than the distance that the stopper member body 510 moves from the third position to the fourth position. When the stopper member (500) is positioned at the third position, the supporting portion (519) may support the shuttle protrusion (435) of the shuttle (431) to restrict the movement of the shuttle (431) positioned at the first position. And when the stopper member (500) moves to the fourth position, the supporting portion (519) disengages from the shuttle protrusion (435), so that the shuttle (431) can move to the second position. **In** addition to the shape that protrudes from the stopper member body (510), the supporting portion (519) can be changed into various other shapes that can contact or engage with the shuttle protrusion (435) or other parts of the shuttle (431).

The stopper member fixing portion (521) may restrict the movement of the stopper member (500) by engaging with one side of the applicator body (300) so that the stopper member (500) maintains the elastically deformed state of the elastic member (541). The stopper member fixing portion (521) includes a pair of fixing hooks (522) that can be engaged with the support fixture (339) of the middle frame (330). The fixing hook (522) may be disengaged from the support fixture (339) by the operation member (345). As shown in (a) of FIG. 16, the fixing hook (522) may be inserted into the support fixture opening (342) of the support fixture (339) and engaged with one side of the support fixture (339). At this time, the stopper member (500) remains fixed in the third position. As shown in (b) of FIG. 16, when the operation member (345) is pressed by the user and advances toward the fixing hook (522), the pressing protrusion (348) of the operation member (345) presses the fixing hook 522. At this time, the fixing hook (522) is biased to be disengaged from the support fixture (339). When the fixing hook (522) is disengaged from the support fixture (339) by the operation member (345), the stopper member (500) is moved to the fourth position by the elastic force of the elastic member (541), as shown in (c) of FIG. 16.

The configuration of the stopper member fixing portion (521) for restraining the movement of the stopper member (500) so that the stopper member (500) maintains the elastically deformed position of the elastic member (541) is not limited to that shown and can be changed in various ways. As another exemplary embodiment, the stopper member fixing portion may be changed to another configuration having a fixing hook that can be engaged with another part of the applicator body (300) in addition to the support (339). As another embodiment, the stopper member fixing portion may be installed to the applicator body (300) to restrict the movement of the stopper member (500). Even in this case, the operation member (345) may operate to release the fixing force of the stopper member (500) by the stopper member fixing portion.

The stopper member locking portion (524) may lock the shuttle (431) in the second position by engaging with the shuttle locking portion (446) of the shuttle (431) that has moved to the second position. The stopper member locking portion (524) includes a locking protrusion (525) that can be inserted into the locking groove (447) of the shuttle locking portion (446). The locking protrusion (525) is provided on one side of the inner surface of the stopper member body (510) so as to protrude into the stopper member opening (512). As shown in (c) of FIG. 16, when the stopper member (500) moves to the fourth position, the locking protrusion (525) may be inserted into the locking groove (447) of the shuttle (431) that has reached the second position. **In** this way, the shuttle member (431) is fixed in the second position by engaging the stopper member locking portion (524) with the shuttle locking portion (446), and the applicator (30) cannot be reused.

The stopper member locking portion (524) and the shuttle locking portion (446) are not limited to the configuration shown and may be changed in various ways. For example, the shape or number of locking protrusions 525 included in the stopper member locking portion (524) may be varied, and the shape or number of locking grooves (447) included in the shuttle locking portion (446) may also be variously changed. Additionally, as another exemplary embodiment, a locking protrusion may be included in the shuttle locking portion and a locking groove into which the locking protrusion may be inserted may be formed in the stopper member locking portion.

The stopper portion (527) is connected to one side of the stopper member body (510) and is disposed in the stopper member opening (512). The stopper portion (527) is in contact with the removing member (610) to maintain the position in which the removing member (610) is coupled to the protective sheet (160) of the sensor unit (100) located in the first position and restrains the movement of removing member (610). The stopper portion (527) has a plurality of stopper portion protrusions (528) that can contact one surface of the removing member (610). An inclined portion (530) is provided at the end of the stopper portion protrusion (528). While the removing member (610) rotates to remove the protective sheet (160), the removing member (610) may come into contact with the inclined portion (530).

The stopper member (500) may restrict the movements of both the shuttle (431) and the removing member (610) in the third position. While moving from the third position to the fourth position, the stopper member (500) may first release the binding force on the removing member (610) and then release the binding force on the shuttle (431). This operation of the stopper member (500) is possible by appropriately setting the length of the support portion (519) and the length of the stopper portion (527) in the movable direction of the stopper member (500). For example, by making the length of the support portion (519) sufficiently long, the shuttle (431) can be released from the support portion (519) after the restraining force on the removing member (610) by the stopper member (500) is completely released. **In** this case, the separation operation of the protective sheet (160) is completed in a state that the sensor unit (100) is located at the first position, and then the shuttle (431) moves so that the sensor unit (100) can move to the second position. The shuttle (431) can start an operation of moving toward the second position during the separation operation of the protective sheet (160) by the removing member (610) depending on the length of the support portion (519) or the length of the stopper portion (527).

In this way, the stopper member (500) can be moved by the operation member (345) to operate the removing unit (600) and the insertion unit (430) sequentially or simultaneously.

The removing unit (600) is installed on the applicator body (300) to separate the protective sheet (160) of the sensor unit (100) from the adhesive layer (155) before the sensor unit (100) reaches the second position and is coupled to the base unit (200). The removing unit (600) includes a removing member (610) rotatably installed on the applicator body (300) to be coupled to the protective sheet (160), and a removing member driver (620) that provides rotational force to the removing member (610).

The removing member (610) may be installed on the applicator body (300) to rotate about a central rotation axis perpendicular to the moving direction of the sensor unit (100). As shown in the drawings, a configuration in which the removing member (610) rotates about a central rotation axis perpendicular to the movable direction of the sensor unit (100) is an exemplary embodiment. As another embodiment, the removing member (610) may be installed so that its rotation center axis intersects or is parallel to the movable direction of the sensor unit (100).

The removing member (610) is provided with a hinge portion (611). The hinge portion (611) is rotatably coupled on the support (318) of the base frame (310), and therefore the removing member (610) can rotate around the hinge portion (611). The hinge portion (611) may be formed to have a structure of being inserted into the support groove (319) of the support (318). The hinge portion (611) is covered with the cover portion (425) of the column member (410) while being placed on the support (318), so that the hinge portion (611) cannot be separated from the support (318) and can remain stably coupled to the support (318). A removing member groove (613) corresponding to the protective sheet opening (162) of the protective sheet (160) is formed on one side of the removing member (610). When the removing member (610) is coupled to the protective sheet (160) of the sensor unit (100) located at the first position, the boss (127) of the sensor unit housing (120) may be located in the removing member groove (613). The removing member (610) may be coupled to the protective sheet (160) through the adhesive member (164) provided on the surface of the protective sheet (160). The adhesive member (164) may be provided on the surface of the removing member (610). In this case, when the surface of the removing member (610) contacts the surface of the protective sheet (160), the adhesive member (164) may be adhered to the protective sheet (160).

The removing member driver (620) includes a removing elastic member (621) that is coupled to the removing member (610) and applies an elastic force to the removing member (610). The removing elastic member (621) is in the form of a torsion spring wound around the hinge portion (611). One side of the removing elastic member (621) is in contact with the applicator body (300) so as to provide the removing member (610) with an elastic force that can rotate the removing member (610).

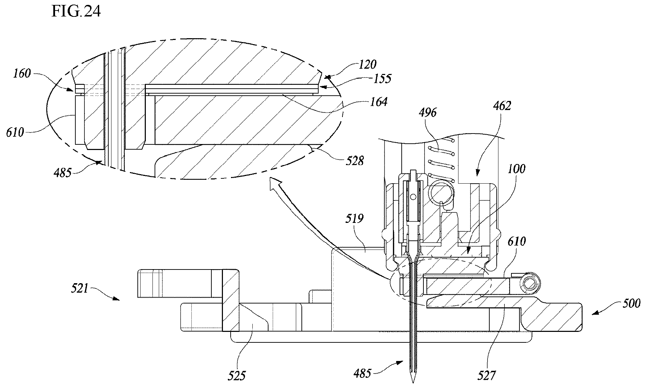

As illustrated in FIG. 24, when the removing member (610) is supported by the stopper portion (527) of the stopper member (500) and placed substantially parallel to the protective sheet (160) of the sensor unit (100) located in the first position, the protective sheet (160) may be coupled with the removing member (610) in a state covering the adhesive layer (155).

Meanwhile, as illustrated in FIG. 24, when the stopper member (500) moves toward the fourth position, the stopper portion (527) moves away from the removing member (610), and the binding force of the removing member (610) by the stopper portion (527) may be released. At this time, the removing member (610) may be rotated by the elastic force of the removing elastic member (621), thereby separating the protective sheet (160) from the adhesive layer (155). While the stopper member (500) moves and the restraining force of the removing member (610) by the stopper portion (527) is released, the removing member (610) contacts the inclined portion (530) provided on the stopper portion protrusion (528) of the stopper portion (527) and can rotate. Accordingly, the removing member (610) can stably separate the protective sheet (160) from the adhesive layer (155) while performing a smoother and more stable rotational movement.

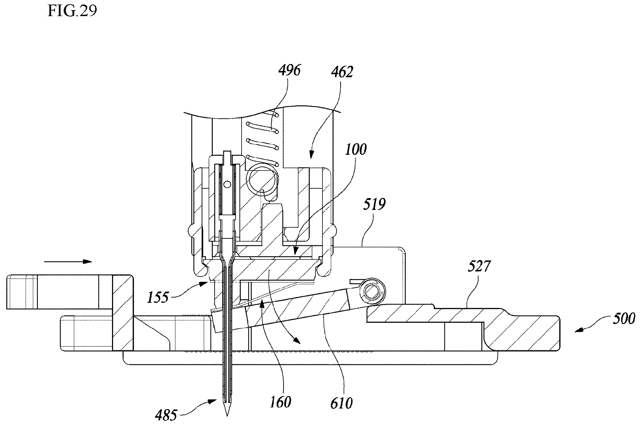

The configuration of the removing member (610) or the removing member driver (620) which constitutes the removing unit (600) is not limited to that shown and can be changed in various ways. For example, the removing member (610) may be rotatably coupled to the applicator body (300) in a manner other than using the hinge portion (611) as illustrated. In addition, the removing member driver (620) may have a spring of another type in addition to the torsion spring-type removing elastic member (621), or may have another configuration capable of providing rotational force to the removing member (610). Additionally, the coupling way of the removing member (610) and the protective sheet (160) may be changed to various other methods other than using the adhesive member (164). As another exemplary embodiment, the removing member (610) may be coupled to the protective sheet (160) in a manner of engaged with the protective sheet (160). Also, as shown in FIG. 29, the removing member (610) may operate to gradually separate the protective sheet (160) from the adhesive layer (155) from one end to the opposite end as its rotation angle increases. Additionally, the rotation angle range of the removing member (610) can be variously set.

The removing unit (600) may operate to complete the separation operation of the protective sheet (160) before the sensor unit (100) moves from the first position. As described above, as the stopper member (500) moves from the third position to the fourth position, the restraining force on the removing member (610) may first be released and then the restraining force on the shuttle (431) may be released. Therefore, it is possible for the removing member (610) to complete the operation of separating the protective sheet (160) before the shuttle (431) moves.

If the sensor unit (100) is moved to the second position in a state that the protective sheet (160) is not completely separated, a problem that the protection sheet (160) may be caught between the sensor unit (100) and the base unit (200), or the sensor unit (100) is coupled to the base unit (200) in a state that the protective sheet (160) is not completely separated may occur. In contrast, the applicator (30) according to the present embodiment completes the separation operation of the protective sheet (160) before the sensor unit (100) moves from the first position, thereby preventing the sensor unit (100) from reaching the base unit (200) in a state that the protective sheet (160) is not separated.

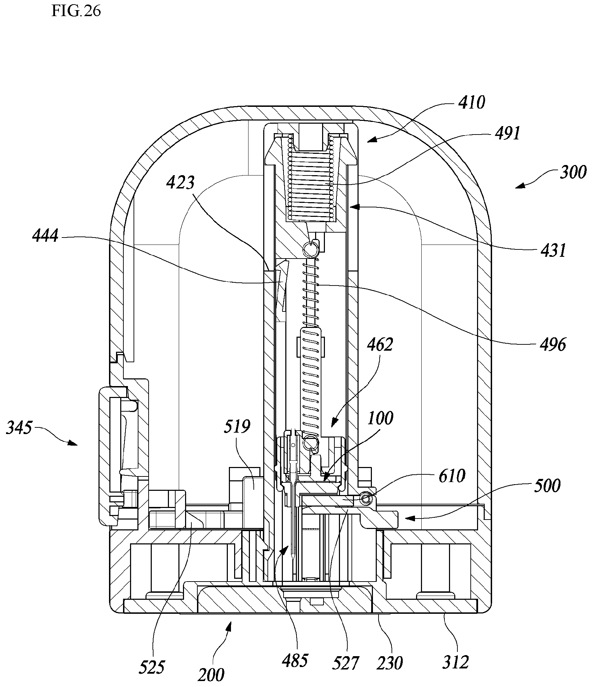

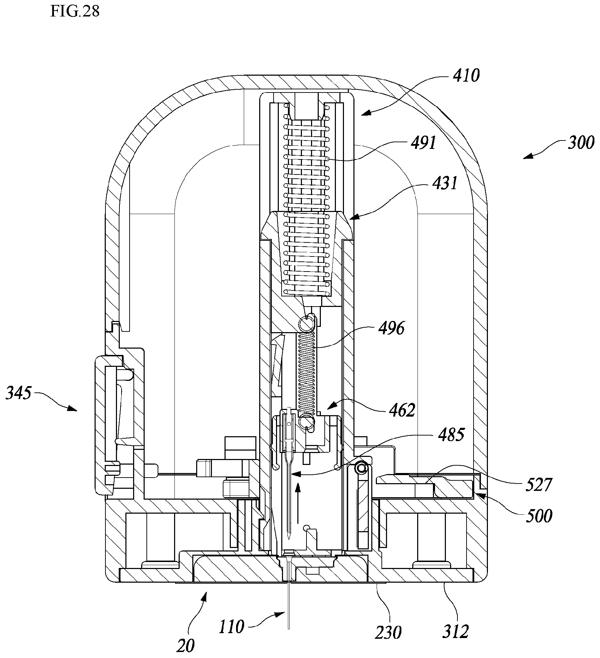

Hereinafter, with reference to FIGS. 26 to 28, a process of attaching the body attachable unit (20) to the skin of a user using the applicator (30) according to an embodiment of the present invention will be described.

As shown in FIG. 26, after the stopper arm (444) of the shuttle (431) is disengaged from the column member detent (423) of the column member (410) and the safety pin (P) is removed, the applicator 30 becomes a state of being operable.

In order to attach the body attachable unit (20) to the skin of a user, The bottom portion (312) of the applicator (30) is positioned on the skin of a user so that the base unit (200) is attached to the skin of a user by the adhesive portion (230). Before the operation member (345) is manipulated, the sensor unit (100) coupled to the carrier (462) is positioned in the first position in a state in which the protective sheet (160) is covering the adhesive layer (155). At this time, the supporting portion (519) of the stopper member (500) supports the shuttle (431) to maintain the position where the shuttle (431) is stopped at the first position. And the stopper portion (527) of the stopper member (500) supports the removing member (610), so that the removing member (610) maintains the position coupled to the protective sheet (160).

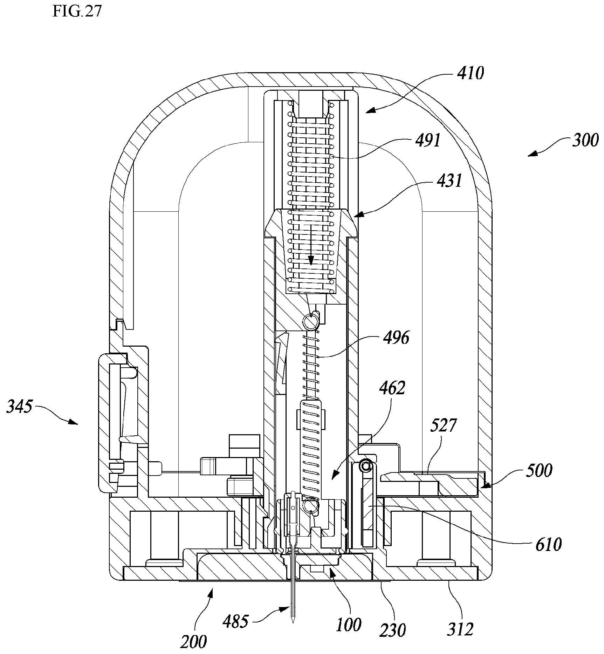

Thereafter, when the user presses the operation member (345), the operation member (345) disengages the stopper member fixing portion (521) of the stopper member (500) from the applicator body (300). At this time, as shown in FIG. 27, the stopper member (500) is moved from the third position to the fourth position by the stopper member driver (540). As the stopper member (500) moves from the third position to the fourth position, the binding force on the removing member (610) is released first, and then the binding force on the shuttle (431) is sequentially released. Accordingly, after the removing member (610) rotates to separate the protective sheet (160) from the adhesive layer (155) of the sensor unit (100), the shuttle (431) moves to the second position. As the shuttle (431) moves to the second position, the needle (485) and the sensor (110) are inserted into the skin of a user, and the sensor unit (100) is attached to the base unit (200) by the adhesive layer (155) so that the attachable unit (20) is assembled. And when the shuttle (431) reaches the second position, the stopper member locking portion (524) of the stopper member (500) engages with the shuttle locking portion (446) of the shuttle (431) so that the shuttle (431) is fixed in the second position, and the shuttle (431) moves the locking hook (360) so that the locking hook (360) is disengaged from the base unit (200).

As previously described, an adhesive layer (155) couples the sensor unit (100) and the base unit (200) and seals between the sensor unit electrical contact portion (146) of the sensor unit (100) and the base unit electrical contact portion (225) of the base unit (200). The adhesive layer (155) seals the electrical connection (250) between the sensor unit (100) and the base unit (200), so that moisture, foreign substances, body fluids, and so on cannot flow into the electrical connection portion (250) between the sensor unit (100) and the base unit (200) in a process of measuring the biosignal by attaching the body attachable unit (20) to the user's skin. Therefore, problems that the body attachment unit (20) malfunctions can be reduced, and stable operation of the body attachable unit (20) is possible.

When the shuttle (431) moves to the second position and the insertion portion (116) of the sensor (110) and the needle (485) are inserted into the skin of a user, the trigger (474) of the carrier (462) comes into contact with the release portion (420) of the column member (410). At this time, the carrier wing (471) of the carrier (462) is elastically deformed, thereby disengaging the carrier latch (477) from the shuttle detent (438).

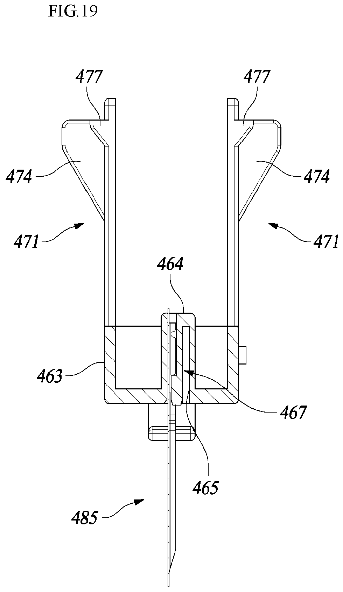

When the carrier (462) is disengaged from the shuttle (431), as shown in FIG. 19, the carrier (462) is moved in a direction away from the skin of a user by the needle release driver (495). At this time, the needle (485) comes out of the skin of a user and the body attachable unit (20) remains attached to the skin of a user by the adhesive portion (230).

As described above, after the insertion portion (116) of the sensor (110) is inserted into the skin, in the process in which the needle (485) is extracted from the user's skin, the retention protrusion (136) of the sensor unit housing (120) contacts the middle portion (113) of the sensor (110), thereby restricting the movement of the insertion portion (116). Therefore, the insertion portion (116) cannot retract in the same direction as the needle (485) and can remain a state of being stably inserted into the skin.

In addition, while the needle (485) retracts in a direction of being away from the skin of the user and is separated from the sensor unit (100), the shuttle bottom portion (440) of the shuttle (431) fixed at the second position maintains the state of being in contact with the sensor unit (100). And, the pressing protrusion (442) of the shuttle (431) maintains of a state of being inserted into the sensor unit groove (138) of the sensor unit (100). Therefore, when the needle (485) is extracted from the skin of the user, the shuttle (431) can support the sensor unit (110) so that the sensor unit (110) cannot be moved, and the insertion portion (116) of the sensor (110) can maintain a status of being stably inserted into the skin.

After the body attachable unit (20) is attached to the skin of a user, the applicator (30) is separated from the body attachable unit (20), and the body attachable unit (20) may measure the biometric information of a user and transmit the measurement information to an external terminal (5), etc.

As described above, the applicator assembly (10) according to an embodiment of the present invention is mounted to the applicator 30 in a state that the adhesive layer (155) of the sensor unit (100) is covered with the protective sheet (160), and therefore, even if it is not used for a long time, the adhesiveness of the adhesive layer (155) does not deteriorate. And, as the user manipulates the operation member (345), the removing unit (600) operates to automatically remove the protective sheet (160) from the sensor unit (100), and the insertion unit (430) operates such that the sensor unit (100) is firmly coupled to the base unit (200) by the adhesive layer (155) and the sensor (110) can be inserted into the user's skin. Therefore, it is convenient to use, and the body attachable unit (20) can be more stably attached to the skin of the user.

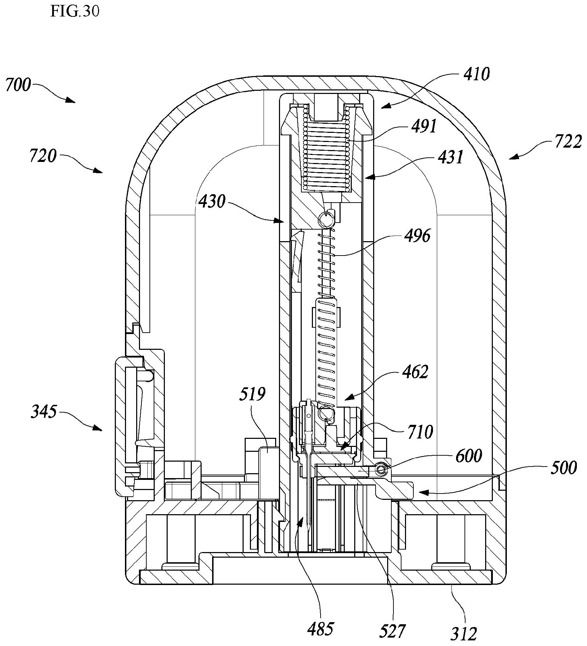

Meanwhile, FIG. 30 shows an applicator assembly according to another embodiment of the present invention.

The applicator assembly (700) shown in FIG. 30 includes a sensor unit (710) including a sensor (110) that is inserted into the skin of the user to measure biometric information, and an applicator (720) which the sensor unit (710) is separably mounted to and is for inserting the sensor (110) into the skin of the user. The sensor unit (710) can be moved from the first position to the second position by the insertion unit (430) installed to the applicator body (722) through the column member (410).

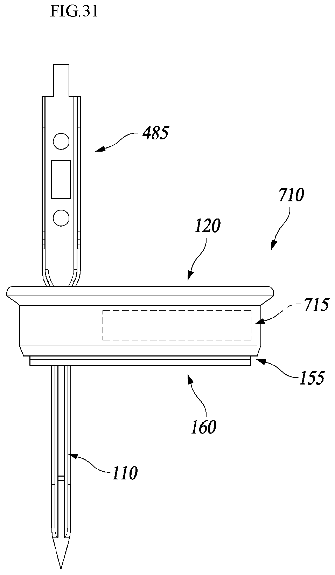

As shown in FIG. 31, the sensor unit (710) includes a sensor (110) inserted into the skin of the user, a sensor unit housing (120) to which the sensor (110) is coupled, an adhesive layer (155) provided at the sensor unit housing (120), a protective sheet (160) covering the adhesive layer (155), and electronic components (715). The electronic components (715) include a circuit board electrically connected to the sensor (110), a process chip installed on the circuit board to convert biometric information measured by the sensor (110) into an electrical signal, and a communication chip for communication with an outside, batteries, and so on. The sensor unit (710) may be attached to the skin of the user by moving from the first position to the second position in a state of being coupled with the needle (485).

The applicator (720) removes the protective sheet (160) from the sensor unit (710) using the removing unit (600) and can move the sensor unit (710) from the first position to the second position using the inserting unit (430).

The sensor (110) may be inserted into the skin of the user at the second position and the sensor unit (710) is can be attached to the user's skin by the adhesive layer (155). After the sensor unit (710) is attached to the skin of the user, the needle (485) is separated from the skin, and the sensor unit (710) is separated from the applicator (720) to measure the biometric information of the user.

The applicator assembly (700) according to the present embodiment can operate without the need for a separate base unit because the sensor unit (710) can be directly attached to the skin of the user and has signal processing and communication functions. That is, the sensor unit (710) can be attached to the skin of the user, measure the biometric information, and transmit the measured biometric information to an external terminal 5 and so on.

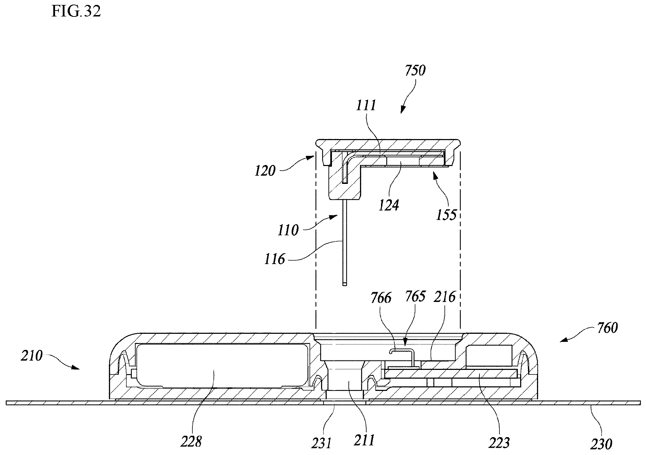

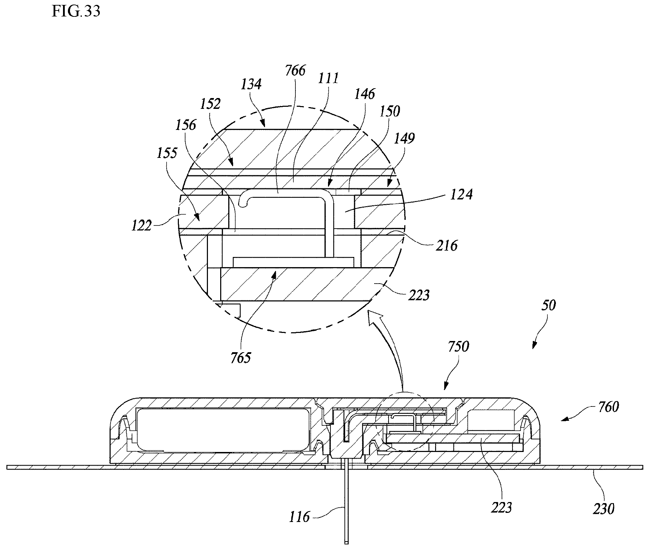

Meanwhile, FIGS. 32 and 33 show other embodiments of a sensor unit and a base unit.

The sensor unit (750) shown in FIGS. 32 and 33 includes a sensor (110) inserted into the skin of the user, a sensor unit housing (120) to which the sensor (110) is coupled, a sensor adhesive portion (149) and an adhesive pad (152) for fixing the sensor (110) to the sensor unit housing (120), an adhesive layer (155) provided at the sensor unit housing (120) to be attached to the base unit (760), a protective sheet covering the adhesive layer (155). The sensor unit (750) has a configuration in which a sensor unit electrical contact portion for electrically connecting the sensor (110) to the base unit (760) is omitted.

The base unit (760) includes a base unit housing (210) to which the sensor unit (750) is coupled, a substrate (223) installed to the inside of the base unit housing (210), a base unit electrical contact (765) electrically connected with the sensor (110) of the sensor unit (750), and a battery (228). An adhesive portion (230) is provided on the surface of the base unit housing (210). The base unit electrical contact (765) contacts the sensor (110) when sensor unit (750) is coupled to the base unit (760). The base unit electrical contact portion (765) is electrically connected to the substrate (223), and a portion of the base unit electrical contact portion 765 may protrude from the contact surface (216) and contact the sensor (110). The base unit electrical contact portion (765) may include terminal portions (766) each contacting electrodes included in the sensor body (111) of the sensor (110). The terminal portion (766) may electrically connect the sensor (110) and the circuit board (223) by contacting the electrode of the sensor body (111). The terminal portion (766) may be formed to be elastically deformable when being in contact with the sensor body (111) so as to stably contact the sensor body (111).

The sensor unit (750) is attached to the base unit (760) by the adhesive layer (155), thereby forming the body attachable unit (50) together with the base unit (760). When the sensor unit (750) is coupled to the base unit (760), the base unit electrical contact portion (765) passes through the adhesive layer hole (156), the through hole (124), and the sensor adhesive hole opening (150) and is connected to the sensor body (111). Accordingly, the sensor (110) may be electrically connected to the base unit (760) through the base unit electrical contact portion (765). At this time, the adhesive layer (155) seals between the sensor (110) and the base unit electrical contact portion (765) to prevent moisture or foreign substances from entering the electrical connection portion between the sensor unit (750) and the base unit (760).

In order to electrically connect the sensor (110) and the base unit (760), the specific configuration, number, and location of the base unit electrical contact portions included in the base unit (760) for electrically connecting the sensor (110) and the base unit (760) may be changed in various ways.

Although the present invention has been described above with preferred examples, the present invention is not limited to the form described and shown above.

For example, in the drawings, the removing member for removing the protective sheet of the sensor unit is installed to be rotatable by receiving rotational force from the removing member driver, and the operation member is operated by the user to operate the removing member driver, but the operation member may be rotatably installed so as to directly rotate the removing member. In this case, the user can rotate the removing member by manually rotating the operation member.

**In** addition, the drawings show that the sensor unit is separably coupled to the carrier coupled with the needle and moves from the first position to the second position, but the sensor unit is separably coupled to the shuttle or a separate member included in the shuttle and can move with the shuttle.

Additionally, the drawings show that the stopper member is installed so that the stopper member can move from the third position to the fourth position by receiving a moving force from the stopper member driver, and the operation member is manipulated by the user to operate the stopper member driver, but it is also possible that the operation member provides a moving force to the stopper member. **In** this case, the operation member can be moved by the user to push the stopper member from the third position to the fourth position.

Further, the drawings show that the operation member operates the insertion unit and the removing unit by moving the stopper member, but the interlocking structure of the operation member and the insertion unit or the interlocking structure of the operation member and the removing unit is not limited to what is illustrated herein. That is, the insertion unit and the removing unit can be installed to operate by different operation members, respectively.

## Claims

1. An applicator (30) for inserting a sensor (110) for measuring biometric information into skin of a user, the applicator (30) comprising:
an applicator body (300);
an insertion unit (430) installed to the applicator body (300) to move a sensor unit (100, 710, 750), which includes the sensor (110), a sensor unit housing (120) to which the sensor is mounted, an adhesive layer (155) provided at the sensor unit housing (120), and a protective sheet (160) covering the adhesive layer (155), from a first position spaced apart from the skin of the user to a second position where the sensor (110) is inserted into the skin of the user;
a removing member (610) to which a portion of the protective sheet (160) is coupled and is rotatably installed to the applicator body (300) to remove the protective sheet (160) from the adhesive layer (155);
an operation member (345) installed to the applicator body (300) to operate the removing member (610) by being manipulated by the user; and
a removing member driver (620) configured to provide rotary force to the removing member (610),
wherein the operation member (345) is configured to operate the removing member driver (620) such that the removing member (610) is rotated by the removing member driver (620).

2. The applicator (30) according to claim 1,
wherein the removing member (610) is configured to be rotatable around a central rotation axis perpendicular to a movable direction of the sensor unit (100, 710, 750).

3. The applicator (30) according to claim 1 or 2,
wherein the removing member (610) is coupled to the protective sheet (160) by an adhesive member (164).

4. The applicator (30) according to any one of the preceding claims,
wherein the removing member driver (620) is coupled with the removing member (610) and configured to apply elastic force to the removing member (610).

5. The applicator (30) according to claim 4, further comprising:
a stopper member (500) installed to the applicator body (300) to restrain movement of the removing member (610) by being contacted with the removing member (610) to maintain a position in which the removing member (610) is coupled with the protective sheet (160) in a state that a removing elastic member is elastically deformed,
wherein the operation member (345) is operated to release restraining force of the removing member (610) caused by the stopper member (500).

6. The applicator (30) according to claim 5,
wherein the stopper member (500) is installed to be movable in a direction of intersecting with a movable direction of the sensor unit (100, 710, 750).

7. The applicator (30) according to claim 6, further comprising:
an elastic member (541) configured to apply elastic force to the stopper member (500) such that the stopper member (500) is movable in a direction of releasing the restraining force of the removing member (610); and
a stopper member fixing portion (521) provided at the stopper member (500) or the applicator body (300) to interlock the stopper member (500) and the applicator body (300) to maintain a position in which the stopper member (500) restrains movement of the removing member (610) in a state that the elastic member (541) is elastically deformed,
wherein the operation member (345) is operated to release fixing force of the stopper member (500) caused by the stopper member fixing portion (521).

8. The applicator (30) according to any one of claims 5 to 7,
wherein the insertion unit (430) comprises:
a shuttle (431) installed to be movable from the first position to the second position together with the sensor unit (100, 710, 750),
a needle (485) coupled with the shuttle (431) to be movable from the first position to the second position together with the shuttle (431), and separably coupled to the sensor unit (100, 710, 750) to be inserted into the skin of the user together with the sensor (110), and
a shuttle driver (490) configured to provide moving force to the shuttle (431) in a direction of moving the first position to the second position, and
wherein movement of the shuttle (431) is restrained by the stopper member (500) at the first position, and the shuttle (431) is movable to the second position by the shuttle driver (490) by releasing restraining force of the stopper member (500) according to movement of the stopper member (500).

9. The applicator (30) according to claim 8,
wherein the stopper member (500) is configured to release restraining force applied to the removing member (610) first, and after completing a separation operation of the protective sheet (160) by the removing member (610), release restraining force applied to the shuttle.

10. The applicator (30) according to any one of the preceding claims,
wherein the removing member (610) is configured to complete a separation operation of the protective sheet (160) before the sensor unit (100, 710, 750) is moved from the first position.

11. The applicator (30) of any one of the preceding claims:
wherein the applicator body (300) is separably coupled to a base unit (200, 760) including a base unit housing (210), and an adhesive portion (230) provided at the base unit housing (210) to be attached to the skin of the user.

12. An applicator assembly (10, 700) comprising:
a sensor unit (100, 710, 750) including a sensor (110), a sensor unit housing (120) to which the sensor is mounted, an adhesive layer (155) provided at the sensor unit housing (120), and a protective sheet (160) covering the adhesive layer (155); and
the applicator (30) according to any one of claims 1 to 11.

## Patentansprüche

1. Applikator (30) zum Einführen eines Sensors (110) zum Messen biometrischer Informationen in die Haut eines Benutzers, wobei der Applikator (30) umfasst:
einen Applikatorkörper (300);
eine Einführeinheit (430), die an dem Applikatorkörper (300) angebracht ist, um eine Sensoreinheit (100, 710, 750), die den Sensor (110), ein Sensoreinheit-Gehäuse (120), an dem der Sensor montiert ist, eine Klebeschicht (155), die an dem Sensoreinheit-Gehäuse (120) vorgesehen ist, und eine Schutzfolie (160), die die Klebeschicht (155) abdeckt, umfasst, von einer ersten Position, die von der Haut des Benutzers beabstandet ist, in eine zweite Position, in der der Sensor (110) in die Haut des Benutzers eingeführt wird, zu bewegen;
ein Entfernungselement (610), an das ein Abschnitt der Schutzfolie (160) gekoppelt ist und das drehbar an dem Applikatorkörper (300) angebracht ist, um die Schutzfolie (160) von der Klebeschicht (155) zu entfernen;
ein Betätigungselement (345), das an dem Applikatorkörper (300) angebracht ist, um das Entfernungselement (610) durch eine Betätigung seitens des Benutzers zu betätigen; und
einen Entfernungselementantrieb (620), der so konfiguriert ist, dass er dem Entfernungselement (610) eine Drehkraft bereitstellt,
wobei das Betätigungselement (345) dazu ausgebildet ist, den Entfernungselementantrieb (620) zu betätigen, so dass das Entfernungselement (610) durch den Entfernungselementantrieb (620) gedreht wird.

2. Applikator (30) nach Anspruch 1,
wobei das Entfernungselement (610) um eine zentrale Drehachse senkrecht zu einer Bewegungsrichtung der Sensoreinheit (100, 710, 750) drehbar ausgebildet ist.

3. Applikator (30) nach Anspruch 1 oder 2,
wobei das Entfernungselement (610) durch ein Klebeelement (164) mit der Schutzfolie (160) gekoppelt ist.

4. Applikator (30) nach einem der vorangehenden Ansprüche,
wobei der Entfernungselementantrieb (620) mit dem Entfernungselement (610) gekoppelt ist und so konfiguriert ist, dass er eine elastische Kraft auf das Entfernungselement (610) ausübt.

5. Applikator (30) nach Anspruch 4, weiterhin umfassend:
ein Stopperelement (500), der an dem Applikatorkörper (300) angebracht ist, um die Bewegung des Entfernungselements (610) zurückzuhalten, indem es mit dem Entfernungselement (610) in Kontakt steht, um eine Position beizubehalten, in der das Entfernungselement (610) mit der Schutzfolie (160) in einem Zustand gekoppelt ist, in dem ein elastisches Entfernungselement elastisch verformt ist,
wobei das Betätigungselement (345) betätigt wird, um die durch das Stopperelement (500) verursachte Rückhaltekraft des Entfernungselements (610) aufzuheben.

6. Applikator (30) nach Anspruch 5,
wobei das Stopperelement (500) so angebracht ist, dass es in einer Richtung bewegbar ist, die sich mit einer Bewegungsrichtung der Sensoreinheit (100, 710, 750) schneidet.

7. Applikator (30) nach Anspruch 6, weiterhin umfassend:
ein elastisches Element (541), das dazu ausgebildet ist, eine elastische Kraft auf das Stopperelement (500) auszuüben, so dass das Stopperelement (500) in einer Richtung bewegbar ist, in der die Rückhaltekraft des Entfernungselements (610) aufgehoben wird; und
der Stopperelement-Fixierabschnitt (521), der an dem Stopperelement (500) oder dem Applikatorkörper (300) vorgesehen ist, um das Stopperelement (500) und das Applikatorgehäuse miteinander zu verriegeln, um eine Position beizubehalten, in der das Stopperelement (500) die Bewegung des Entfernungselements (610) in einem Zustand, in dem das elastische Element (541) elastisch verformt ist, zurückhält,
wobei das Betätigungselement (345) betätigt wird, um die durch den Stopperelement-Fixierabschnitt (521) verursachte Fixierkraft des Stopperelements (500) aufzuheben.

8. Applikator (30) nach einem der Ansprüche 5 bis 7,
wobei die Einführeinheit (430) umfasst:
einen Schlitten (431), der so angebracht ist, dass er zusammen mit der Sensoreinheit (100, 710, 750) von der ersten Position in die zweite Position bewegbar ist,
eine Nadel (485), die mit dem Schlitten (431) so gekoppelt ist, dass sie zusammen mit dem Schlitten (431) von der ersten Position in die zweite Position bewegbar ist, und die trennbar mit der Sensoreinheit (100, 710, 750) so gekoppelt ist, dass sie zusammen mit dem Sensor (110) in die Haut des Benutzers eingeführt werden kann, und
einen Schlittenantrieb (490), der dazu ausgebildet ist, dem Schlitten (431) eine Bewegungskraft in Richtung der Bewegung von der ersten Position in die zweite Position bereitzustellen, und
wobei die Bewegung des Schlittens (431) an der ersten Position durch das Stopperelement (500) zurückgehalten wird und der Schlitten durch den Schlittenantrieb (490) in die zweite Position bewegbar ist, indem die Rückhaltekraft des Stopperelements (500) entsprechend der Bewegung des Stopperelements aufgehoben wird.

9. Applikator (30) nach Anspruch 8,
wobei das Stopperelement (500) dazu ausgebildet ist, zunächst die auf das Entfernungselement ausgeübte Rückhaltekraft aufzuheben und nach Abschluss eines Trennvorgangs der Schutzfolie (160) durch das Entfernungselement (610) die auf den Schlitten ausgeübte Rückhaltekraft aufzuheben.

10. Applikator (30) nach einem der vorangehenden Ansprüche,
wobei das Entfernungselement (610) dazu ausgebildet ist, einen Trennvorgang der Schutzfolie (160) abzuschließen, bevor die Sensoreinheit (100, 710, 750) aus der ersten Position bewegt wird.

11. Applikator (30) nach einem der vorangehenden Ansprüche:
wobei der Applikatorkörper (300) trennbar mit einer Basiseinheit (200, 760) gekoppelt ist, die ein Basiseinheit-Gehäuse (210) und einen Klebeabschnitt (230) umfasst, der an dem Basiseinheit-Gehäuse (210) vorgesehen ist und an der Haut des Benutzers befestigt werden kann.

12. Applikatoranordnung (10, 700), umfassend:
eine Sensoreinheit (100, 710, 750), die einen Sensor (110), ein Sensoreinheit-Gehäuse (120), an dem der Sensor montiert ist, eine Klebeschicht (155), die an dem Sensoreinheit-Gehäuse (120) vorgesehen ist, und eine Schutzfolie (160), die die Klebeschicht abdeckt (155), beinhaltet; und
den Applikator (30) nach einem der Ansprüche 1 bis 11.

## Revendications

1. Applicateur (30) pour l'insertion d'un capteur (110) destiné à la mesure d'informations biométriques dans la peau d'un utilisateur, ledit applicateur (30) comprenant :
un corps d'applicateur (300) ;
une unité d'insertion (430) montée sur le corps de l'applicateur (300) pour déplacer une unité de capteur (100, 710, 750) comprenant le capteur (110), un boîtier d'unité de capteur (120) sur lequel est monté le capteur, une couche adhésive (155) disposée sur le boîtier d'unité de capteur (120) et une feuille de protection (160) recouvrant la couche adhésive (155), d'une première position espacée de la peau de l'utilisateur à une deuxième position où le capteur (110) est inséré dans la peau de l'utilisateur ;
un élément de retrait (610) auquel une partie de la feuille de protection (160) est raccordée et est disposée de manière rotative sur le corps de l'applicateur (300) pour retirer la feuille de protection (160) de la couche adhésive (155) ;
un élément d'actionnement (345) monté sur le corps de l'applicateur (300) pour actionner l'élément de retrait (610) en étant manipulé par l'utilisateur ; et
un entraînement d'élément de retrait (620) prévu pour appliquer une force de rotation à l'élément de retrait (610),
où l'élément de retrait (345) est prévu pour actionner le dispositif d'entraînement d'élément de retrait (620) de manière à faire pivoter l'élément de retrait (610) sous l'effet du dispositif d'entraînement d'élément de retrait (620).

2. Applicateur (30) selon la revendication 1,
où l'élément de retrait (610) est prévu pour être pivotant autour d'un axe de rotation central perpendiculaire à une direction de déplacement de l'unité de capteur (100, 710, 750).

3. Applicateur (30) selon la revendication 1 ou la revendication 2,
où l'élément de retrait (610) est raccordé à la feuille de protection (160) par un élément adhésif (164).

4. Applicateur (30) selon l'une des revendications précédentes,
où l'entraînement d'élément de retrait (620) est raccordé à l'élément de retrait (610) et prévu pour appliquer une force élastique sur l'élément de retrait (610).

5. Applicateur (30) selon la revendication 4, comprenant en outre :
un élément de butée (500) monté sur le corps d'applicateur (300) pour limiter le déplacement de l'élément de retrait (610) en étant mis en contact avec l'élément de retrait (610) afin de maintenir une position où l'élément de retrait (610) est raccordé à la feuille de protection (160) dans un état où un élément élastique de retrait est déformé élastiquement,
l'élément d'actionnement (345) étant actionné pour relâcher la force de retenue de l'élément de retrait (610) due à l'élément de butée (500).

6. Applicateur (30) selon la revendication 5,
où l'élément de butée (500) est monté de manière à être mobile dans une direction croisant la direction de déplacement de l'unité de capteur (100, 710, 750).

7. Applicateur (30) selon la revendication 6, comprenant en outre :
un élément élastique (541) prévu pour appliquer une force élastique à l'élément de butée (500) de sorte que l'élément de butée (500) est déplaçable dans une direction de relâchement de la force de retenue de l'élément de retrait (610) ; et
une partie de fixation d'élément de butée (521) prévue sur l'élément de butée (500) ou le corps d'applicateur (300) pour verrouiller l'élément de butée (500) et le corps d'applicateur (300) afin de maintenir une position où l'élément de butée (500) limite le déplacement de l'élément de retrait (610) dans un état où l'élément élastique (541) est déformé élastiquement,
l'élément d'actionnement (345) étant actionné pour relâcher la force de fixation de l'élément de butée (500) due à la partie de fixation d'élément de butée (521).

8. Applicateur (30) selon l'une des revendications 5 à 7,
où l'unité d'insertion (430) comprend :
une navette (431) montée de manière à être déplaçable de la première position à la deuxième position avec l'unité de capteur (100, 710, 750),
une aiguille (485) raccordée à la navette (431) de manière à être déplaçable de la première position à la deuxième position avec la navette (431), et raccordée de manière séparable à l'unité de capteur (100, 710, 750) pour être insérée dans la peau de l'utilisateur avec le capteur (110), et
un entraînement de navette (490) prévu pour appliquer une force de déplacement à la navette (431) dans une direction de déplacement de la première position vers la deuxième position, et
où le déplacement de la navette (431) est limité par l'élément de butée (500) au niveau de la première position, et la navette (431) peut être déplacée vers la deuxième position par le dispositif d'entraînement de navette (490) relâchant une force de limitation de l'élément de butée (500) en fonction du déplacement de l'élément de butée (500).

9. Applicateur (30) selon la revendication 8,
où l'élément de butée (500) est prévu pour relâcher d'abord la force de retenue appliquée à l'élément de retrait (610), puis, à l'issue d'une action de séparation de la feuille de protection (160) par l'élément de retrait (610), relâcher la force de retenue appliquée à la navette.

10. Applicateur (30) selon l'une des revendications précédentes,
où l'élément de retrait (610) est prévu pour exécuter une action de séparation de la feuille de protection (160) avant le déplacement de l'unité de capteur (100, 710, 750) depuis la première position.

11. Applicateur (30) selon l'une des revendications précédentes :
où le corps d'applicateur (300) est raccordé de manière séparable à une unité de base (200, 760) comprenant un boîtier d'unité de base (210), et une partie adhésive (230) disposée sur le boîtier d'unité de base (210) pour être fixée contre la peau de l'utilisateur.

12. Ensemble applicateur (10, 700), comprenant :
une unité de capteur (100, 710, 750) comprenant un capteur (110), un boîtier d'unité de capteur (120) sur lequel le capteur est monté, une couche adhésive (155) disposée sur le boîtier d'unité de capteur (120), et une feuille de protection (160) recouvrant la couche adhésive (155) ; et
l'applicateur (30) selon l'une des revendications 1 à 11.
